# EUROPEAN PATENT APPLICATION

(11) **EP 2 684 933 A1**
(43) Date of publication of application: **15.01.2014**
(21) Application number: 12754673.7
(22) Date of filing: 05.03.2012
(51) Int. Cl.: C09K 11/06, C07D 213/16, C07F 15/00, H01L 51/50

(54) **PHOSPHORESCENT MATERIAL, PROCESS FOR PRODUCING PHOSPHORESCENT MATERIAL, AND PHOSPHORESCENT ELEMENT**

(30) Priority: 10.03.2011 JP 2011053021
(71) Applicant: Kyushu University, Fukuoka-shi, Fukuoka 812-8581 (JP); LINTEC Corporation, Tokyo 173-0001 (JP)
(72) Inventor: TANEDA Masatsugu, Fukuoka-shi Fukuoka 812-8581 (JP); ADACHI Chihaya, Fukuoka-shi Fukuoka 812-8581 (JP); YASUDA Takuma, Fukuoka-shi Fukuoka 812-8581 (JP); NAKATA Manabu, Kadoma-shi Osaka 571-8501 (JP); NAKATA Yasukazu, Tokyo 173-0001 (JP)
(74) Representative: MERH-IP Matias Erny Reichl Hoffmann
(86) International application number: PCT/JP2012/055513
(87) International publication number: WO 2012/121189

(57) **Abstract**

Provided are a phosphorescent material which is excellent in horizontal orientation and the like when a thin film is formed, a process for efficiently producing the phosphorescent material, and a light emitting element using the phosphorescent material.

The present invention provides a phosphorescent material represented by the following general formula (1), a process for producing the phosphorescent material, and a light emitting element using the phosphorescent material, wherein the phosphorescent material has a straight-chain conjugated structure, a 2-phenylpyridine ligand, a central metal and a β-diketone-type ligand,

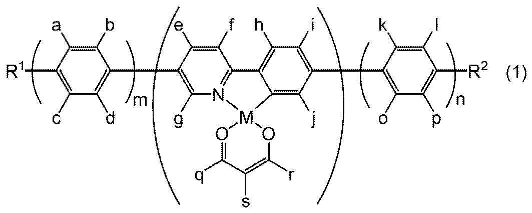

In the general formula (1), end substituents R¹ and R² each is a hydrogen atom, an alkyl group having 1 to 20 carbon atoms, or the like, substituents a to l and o to s each is a hydrogen atom or the like, the central metal M is platinum or the like, and repetition numbers m and n each is an integer of 0 to 4.

## Description

### BACKGROUND

### Technical Field

The present invention relates to a phosphorescent material, a process for producing a phosphorescent material, and a phosphorescent element. Particularly, the present invention relates to a phosphorescent material which is excellent in horizontal orientation and the like when formed into a film, a process for producing a phosphorescent material, and a phosphorescent element.

### Related Art

Use of phosphorescence, in place of fluorescent light, has been proposed as an attempt to improving light emission efficiency of organic electroluminescence elements (organic EL elements).

That is, it is anticipated that when in a light emitting layer of the organic EL element, a phosphorescent material is included in a predetermined amount with respect to a host material as a main component, and an excited triplet state of the phosphorescent material is principally used, high light emission efficiency is achieved. This is because an excited singlet state and the excited triplet state, which are different in spin multiplicity, may be generated in a ratio of 1 : 3 when an electron and hole are recombined within the organic EL element.

Therefore, about only 25% of excitons (100%) can be used when fluorescent light, which is light emitted at the time of going back to a ground state from the excited singlet state, is used, while many excitons can be used when phosphorescence, which is light emitted at the time of going back to the ground state from the excited triplet state. That is, occurrence of intersystem crossing causes a change from the excited singlet state to the excited triplet state, so that approximately 100% of the obtained excitons can be used, and therefore improvement of light emission efficiency is expected.

Thus, for improving light emission efficiency as described above, there has been proposed an organic EL element including a light emitting layer having a carbazole compound as a main component and containing a phosphorescent iridium complex material in a predetermined amount (see, for example, Patent Document 1).

More specifically, the organic EL element is an organic EL element formed by sequentially laminating an anode, a hole transport layer, a light emitting layer containing a phosphorescent iridium complex material, an electron transport layer including an organic compound, and a cathode, wherein the light emitting layer has a carbazole compound as a main component and contains the iridium complex material in an amount of 0.5 to 8% by weight.

As a typical phosphorescent iridium complex material, tris(2-phenylpyridine)iridium (hereinafter, referred to Ir(PPY)₃ in some cases) represented by the following formula (A) is disclosed.

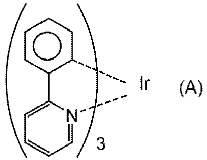

For improving a light emission property and the life of an element, there have been proposed a phosphorescent organic metal complex having a predetermined structure and a light emitting element containing the phosphorescent organic metal complex in a light emitting layer (see, for example, Patent Document 2).

More specifically, there has been proposed a light emitting element (organic EL element) containing in a light emitting layer an phosphorescent organic metal complex in which β-dicarbonyl at an end of a long carbon chain represented by the following general formula (B) and two 2-phenylpyridine molecules are coordinated to a platinum atom or the like (M, M') and β-dicarbonyl at the other end of the carbon chain has a similar coordination structure.

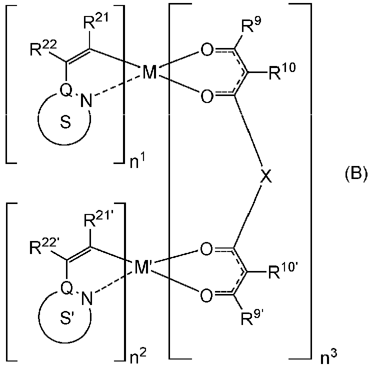

(In the general formula (B), rings S and S' each independently represents a nitrogen-containing aromatic heterocyclic ring which may have a substituent; Qs each independently is an atom which forms the ring S or S', and represent a carbon atom or a nitrogen atom; R²¹, R²², R²¹' and R²²' each independently represents a hydrogen atom or a substituent of a vinylene group or the like; R⁹ and R⁹' each independently represents an alkyl group, an aryl group or the like; R¹⁰ and R¹⁰' each independently represents a hydrogen atom, an alkyl group, an aryl group or the like; M and M' each independently represents an iridium atom or a platinum atom; n¹ to n³ each independently represents an integer of 1 or 2; and X represents a linking group.)

There have been proposed a phosphorescent pigment which has a high light emission luminance, is firm and has good solubility, and an organic electroluminescence element containing the phosphorescent pigment in a light emitting layer (see, for example, Patent Document 3).

More specifically, there has been proposed a light emitting element (organic EL element) containing in a light emitting layer a phosphorescent organic metal complex represented by the following general formula (C).

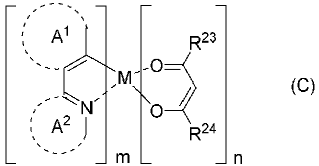

(In the general formula (C), M is a divalent or trivalent metal atom, A¹ is an aromatic ring or a heterocyclic ring, A² is a heterocyclic ring, R²³ and R²⁴ are the same or different and each is an alkyl group or an aryl group, at least one of R²³ and R²⁴ is an aryl group, m and n each is 1 or 2, and the sum of m and n is a valence number (2 or 3) of the metal atom.)

### Citation List

### Patent Documents

[Patent Document 1] JP2001313178A (claims and the like)
[Patent Document 2] JP2007277170A (claims and the like)
[Patent Document 3] JP2008222635A (claims and the like)

### SUMMARY

However, the phosphorescent iridium complex material disclosed in Patent Document 1 and the phosphorescent organic metal complexes disclosed in Patent Documents 2 and 3 have such a problem that the horizontal orientation when they are formed into a film is inadequate, and transition moments are not equalized, so that polarizability is poor, and an organic EL element having a high luminance cannot be obtained yet.

The phosphorescent iridium complex material disclosed in Patent Document 1 and the phosphorescent organic metal complexes disclosed in Patent Documents 2 and 3 also have such a problem that when they are used in the light emitting material of the organic EL element, a range of the adding quantity of the material that can be blended with a carbazole compound or the like as a host material, which is a main component, is narrow.

Therefore, when a past phosphorescent material is used, there is such a problem that it is difficult to stably form the light emitting layer in the organic EL element, and the light emission property and the life of the element are still poor.

Accordingly, in view of the above situations, the present inventors have found that by including at least a straight-chain conjugated structure, a 2-phenylpyridine ligand (including a ligand of a 2-phenylpyridine derivative; the same hereinafter), a central metal (referred to as a coordination metal in some cases; the same hereinafter) and a β-diketone-type ligand (including an acetyl acetate ligand and a ligand of an acetyl acetate derivative; the same hereinafter) in a molecule of a phosphorescent material, phosphorescence having high polarizability and a high luminance is obtained in a horizontal direction with respect to a substrate when the material is formed into a film having a predetermined thickness and light is applied thereto at a predetermined angle (in the 90° direction), and the material can be added in a wide range of the mixed quantity to a host material as a main component in a light emitting layer of an organic EL element, thus leading to completion of the present invention.

That is, an object of the present invention is to provide a phosphorescent material which is excellent in horizontal orientation and the like when formed into a film, a process for efficiently producing the phosphorescent material, and a phosphorescent element using the phosphorescent material.

According to the invention of the present application, there is provided a phosphorescent material represented by the following general formula (1), wherein the phosphorescent material has a straight-chain conjugated structure (partially including a ring structure in some cases; the same hereinafter), a 2-phenylpyridine ligand, a central metal and a β-diketone-type ligand, and the problems described above can be solved.

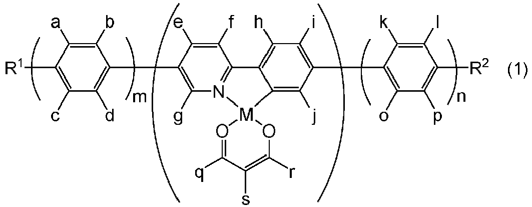

(In the general formula (1), end substituents R¹ and R² are each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group (including a carbazole group), substituents a to l and o to s are each independently a hydrogen atom, a substituted or unsubstituted alkyl group having 1 to 20 carbon atoms, a substituted or unsubstituted aryl group having 6 to 20 carbon atoms, or a halogen atom, the central metal M is platinum (Pt), iridium (Ir), nickel (Ni), copper (Cu) or gold (Au), repetition numbers m and n are each independently an integer of 0 to 4, and m + n is an integer of 1 or greater.)

That is, by constructing the phosphorescent material as described above, an excellent horizontal orientation and the like can be achieved when a film is formed using the phosphorescent material.

Therefore, when such a phosphorescent material is used as a dopant material of a light emitting layer in an organic EL element, and light is applied at a predetermined angle (in the normal line direction, the 90° direction with respect to a substrate), phosphorescence having high polarizability and a high luminance can be obtained, and the material can be added in a wide range of the mixed quantity to a host material as a main component.

Whether phosphorescence is emitted or not can be determined by a light emission life in the phosphorescent material as measured using, for example, Quantaurus-Tau (manufactured by Hamamatsu Photonics K.K.) which is a small fluorescent light life measuring device.

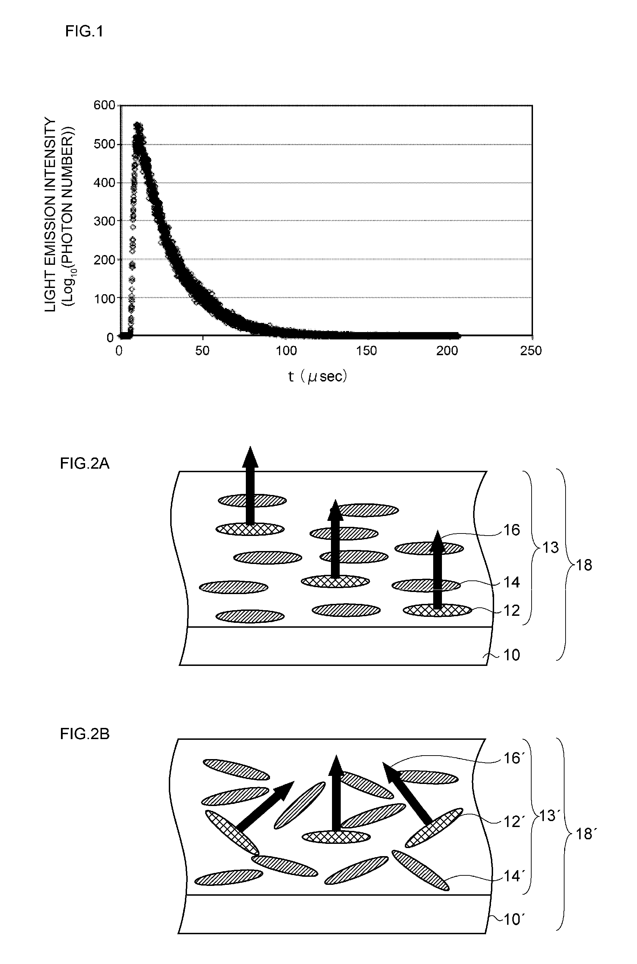

That is, as shown in FIG. 1, a light emission spectrum of phosphorescence is measured, and when a predetermined light emission intensity (Log₁₀ (photon number)) is maintained in an order of µsec or more, it can be determined that the resulting emitted light is phosphorescence.

When the phosphorescent material of the invention of the present application is constructed, R¹ and R², or any one thereof, are preferably a tertiary butyl group.

By constructing the phosphorescent material as described above, association of molecules of the phosphorescent material can be effectively prevented, and when it is used as a dopant material of the light emitting layer of the organic EL element, a light emission property with a further high luminance can be achieved.

When the phosphorescent material of the invention of the present application is constructed, the Stokes shift is set to preferably a value of 100 nm or more.

By constructing the phosphorescent material as described above, phosphorescence having a further high luminance can be efficiently obtained when the phosphorescent material is excited to emit light.

When the phosphorescent material of the invention of the present application is constructed, the phosphorescent material represented by the general formula (1) is preferably at least one of compounds represented by the following formulae (2) to (10).

By constructing the phosphorescent material as described above, phosphorescence which has a further high luminance and is stable can be obtained, and good heat resistance and good dispersibility in the host material can be achieved.

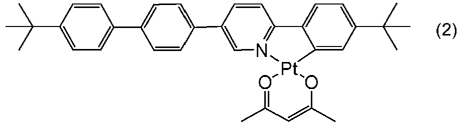

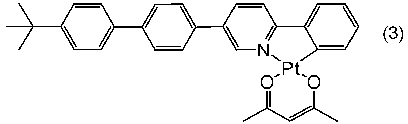

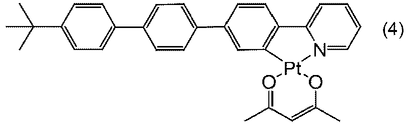

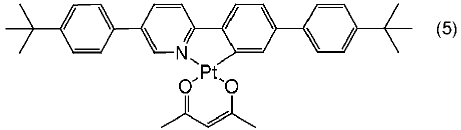

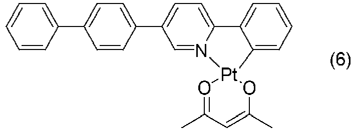

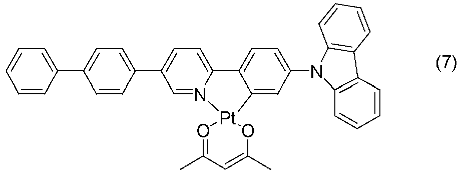

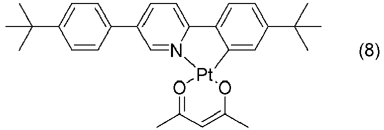

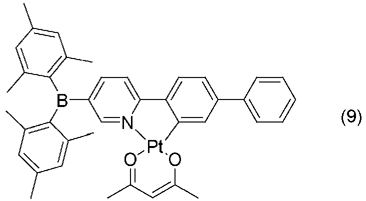

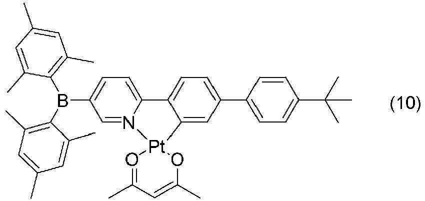

Another aspect of the invention of the present application is a process for producing a phosphorescent material represented by the above general formula (1) and having a straight-chain conjugated structure, a 2-phenylpyridine ligand, a central metal and a β-diketone-type ligand, wherein the process includes steps of: providing a binuclear complex represented by the following general formula (11); and acetylacetonating the binuclear complex.

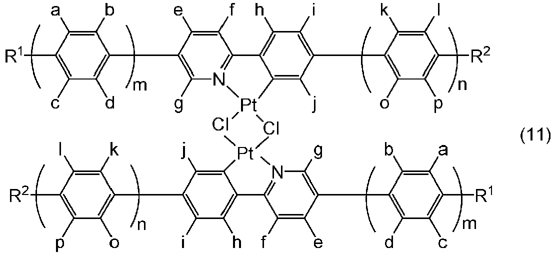

(In the general formula (11), a plurality of end substituents R¹ and R² is each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group, a plurality of substituents a to l and o to s is each independently a hydrogen atom, a substituted or unsubstituted alkyl group having 1 to 20 carbon atoms, a substituted or unsubstituted aryl group having 6 to 20 carbon atoms, or a halogen atom, a plurality of central metals M is each independently platinum (Pt), iridium (Ir), nickel (Ni), copper (Cu) or gold (Au), a plurality of repetition numbers m and n is each independently an integer of 0 to 4, and m + n is an integer of 1 or greater.)

By practicing the process for producing as described above, a phosphorescent material, which shows an excellent horizontal orientation and the like when formed into a film, can be efficiently produced.

Therefore, by using such a phosphorescent material as a dopant material of the light emitting layer in the organic EL element, high-luminance phosphorescence including a large amount of optical components having high polarizability can be obtained at relatively low costs.

When the process for producing a phosphorescent material according to the present invention is practiced, preferably the process includes a step of obtaining the binuclear complex represented by the general formula (11) by synthesizing a straight-chain aryl compound including a pyridine structure, followed by performing a heat treatment in the presence of potassium tetrachloroplatinate and acetic acid.

By synthesizing the binuclear complex represented by the general formula (11), a phosphorescent material, which is excellent in horizontal orientation and the like when formed into a film, can be efficiently produced.

Still another aspect of the invention of the present application is a light emitting element which includes a light emitting layer or a plurality of organic thin film layers including the light emitting layer between a pair of electrodes including an anode and a cathode, wherein the light emitting layer contains a host material as a main component and, as a dopant material, a phosphorescent material represented by the general formula (1) and having a straight-chain conjugated structure, a 2-phenylpyridine ligand, a central metal and a β-diketone-type ligand.

By constructing the light emitting element as described above, high-luminance phosphorescence including a large amount of light emission components having high polarizability can be obtained.

When the light emitting element of the invention of the present application is constructed, the mixed quantity of the phosphorescent material is set to preferably a value ranging from 0.1 to 20% by weight based on the total amount of the light emitting layer.

By constructing the light emitting element as described above, high-luminance phosphorescence including a large amount of optical components having high polarizability can be stably obtained.

### BRIEF DESCRIPTION OF DRAWINGS

FIG. 1 is a view showing a relationship between a light emission time t (µsec) and a light emission intensity in a phosphorescent emission spectrum;
FIG. 2A is a view provided for explaining the horizontal orientation when a thin film is formed using a phosphorescent material of the invention of the present application as a dopant material, and FIG. 2B is a view provided for explaining the non-horizontal orientation when a thin film is formed using a past phosphorescent material as a dopant material;
FIG. 3 is a view of a device for measuring polarizability of the phosphorescent material of the invention of the present application (incident angle to thin film: 90°);
FIG. 4 is a view of a device for measuring non-polarizability of the phosphorescent material of the invention of the present application (incident angle to thin film: 45°);
FIG. 5 is a sectional view of a basic organic EL element;
FIG. 6 is a sectional view of a modification of an organic EL element including an electron injection layer;
FIG. 7 is a sectional view of a modification of an organic EL element including a hole injection layer;
FIG. 8 is a sectional view of a modification of another organic EL element;
FIG. 9 is a sectional view of a modification of still another organic EL element;
FIG. 10 is a NMR chart of a phosphorescent material (example 1);
FIG. 11 is a FT-IR chart of the phosphorescent material (example 1);
FIG. 12 is a phosphorescence emission spectrum of the phosphorescent material (example 1) which is obtained by the measuring device shown in FIG. 3;
FIG. 13 is a phosphorescence emission spectrum of the phosphorescent material (example 1) which is obtained by the measuring device shown in FIG. 4;
FIG. 14 is a phosphorescence emission spectrum of the phosphorescent material (example 1);
FIG. 15 is a NMR chart of a phosphorescent material (example 2);
FIG. 16 is a FT-IR chart of the phosphorescent material (example 2);
FIG. 17 is a phosphorescence emission spectrum of the phosphorescent material (example 2) which is obtained by the measuring device shown in FIG. 3;
FIG. 18 is a phosphorescence emission spectrum of the phosphorescent material (example 2) which is obtained by the measuring device shown in FIG. 4;
FIG. 19 is a phosphorescence emission spectrum of the phosphorescent material (example 2);
FIG. 20 is a view provided for explaining a relationship between a voltage and a current density in an organic EL element using the phosphorescent material (example 2);
FIG. 21 is a view provided for explaining a relationship between a current density and external quantum efficiency in the organic EL element using the phosphorescent material (example 2);
FIG. 22 is a light emission spectrum in the organic EL element using the phosphorescent material (example 2);
FIG. 23 is a NMR chart of a phosphorescent material (example 3);
FIG. 24 is a FT-IR chart of the phosphorescent material (example 3);
FIG. 25 is a phosphorescence emission spectrum of the phosphorescent material (example 3) which is obtained by the measuring device shown in FIG. 3;
FIG. 26 is a phosphorescence emission spectrum of the phosphorescent material (example 3) which is obtained by the measuring device shown in FIG. 4;
FIG. 27 is a phosphorescence emission spectrum of the phosphorescent material (example 3);
FIG. 28 is a NMR chart of a phosphorescent material (example 4);
FIG. 29 is a FT-IR chart of the phosphorescent material (example 4);
FIG. 30 is a phosphorescence emission spectrum of the phosphorescent material (example 4) which is obtained by the measuring device shown in FIG. 3;
FIG. 31 is a phosphorescence emission spectrum of the phosphorescent material (example 4) which is obtained by the measuring device shown in FIG. 4;
FIG. 32 is a phosphorescence emission spectrum of the phosphorescent material (example 4);
FIG. 33 is a NMR chart of a phosphorescent material (example 5);
FIG. 34 is a FT-IR chart of the phosphorescent material (example 5);
FIG. 35 is a NMR chart of a phosphorescent material (example 6);
FIG. 36 is a FT-IR chart of the phosphorescent material (example 6);
FIG. 37 is a NMR chart of a phosphorescent material (example 7);
FIG. 38 is a FT-IR chart of the phosphorescent material (example 7);
FIG. 39 is a NMR chart of a phosphorescent material (example 8);
FIG. 40 is a FT-IR chart of the phosphorescent material (example 8);
FIG. 41 is a NMR chart of a phosphorescent material (example 9);
FIG. 42 is a FT-IR chart of the phosphorescent material (example 9);
FIG. 43 is a phosphorescence emission spectrum of the phosphorescent material (comparative example 1) which is obtained by the measuring device shown in FIG. 3;
FIG. 44 is a phosphorescence emission spectrum of the phosphorescent material (comparative example 1) which is obtained by the measuring device shown in FIG. 4; and
FIG. 45 is a phosphorescence emission spectrum of the phosphorescent material (comparative example 1).

### DETAILED DESCRIPTION

### [First Embodiment]

The first embodiment of the present invention is a phosphorescent material represented by the general formula (1), wherein the phosphorescent material has a straight-chain conjugated structure, a 2-phenylpyridine ligand, a central metal and a β-diketone-type ligand.

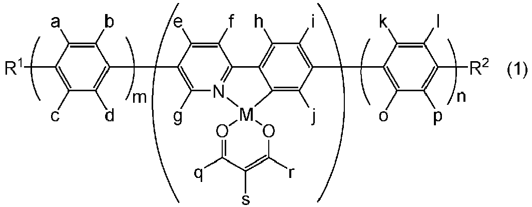

(In the general formula (1), end substituents R¹ and R² are each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group, substituents a to l and o to s are each independently a hydrogen atom, a substituted or unsubstituted alkyl group having 1 to 20 carbon atoms, a substituted or unsubstituted aryl group having 6 to 20 carbon atoms, or a halogen atom, the central metal M is platinum (Pt), iridium (Ir), nickel (Ni), copper (Cu) or gold (Au), repetition numbers m and n are each independently an integer of 0 to 4, and m + n is an integer of 1 or greater.)

An aspect of the phosphorescent material which is the first embodiment of the present invention will be specifically described below with reference to the drawings as appropriate.

### 1. Phosphorescent material represented by general formula (1)

### (1) Basic structure of phosphorescent material

The phosphorescent material of the first embodiment is a compound represented by the above general formula (1), and has as a basic structure a straight-chain conjugated structure (partially including a ring structure in some cases), a 2-phenylpyridine ligand, a central metal and a β-diketone-type ligand (acetylacetonate ligand).

That is, since the phosphorescent material has a straight-chain conjugated structure containing a 2-phenylpyridine ligand in a molecule, the horizontal orientation of molecules 12 of the phosphorescent material can be significantly improved when a thin film containing the phosphorescent material is formed as shown in FIG. 2A.

It is thought that the molecule 12 of the phosphorescent material defines the arrangement of not only itself but also molecules 14 of a host material contained in a light emitting layer 13 to some extent, so that their horizontal orientation is improved as well.

Therefore, when molecules 12 of the phosphorescent material are excited to emit light, travelling directions of phosphorescence 16 are equalized to a predetermined direction (normal line direction of thin film), i.e. transition moments in molecules 12 of the phosphorescent material are equalized, so that phosphorescence having high light emission luminance can be stably obtained.

This is indicated by FIG. 2A where travelling directions of emitted phosphorescence 16 lie in a fixed direction.

However, it has been separately confirmed that phosphorescence having a considerable light emission luminance is stably obtained even if the arrangement of molecules 14 of the host material contained in the light emitting layer 13 remains random without changing in response to the horizontal orientation of molecules 12 of the phosphorescent material.

The straight-chain conjugated structure should have a plurality of aryl rings arranged in a line while including a 2-phenylpyridine backbone.

Therefore, preferably a linear structure including 3 to 10 aryl rings, more preferably a linear structure including 4 to 8 aryl rings is formed by, for example, adjusting the type of a raw material, a synthesis reaction and the like, and appropriately changing the value of m + n on the precondition that a ring structure containing a 2-phenylpyridine ligand is included.

On the other hand, if the phosphorescent material does not have a straight-chain conjugated structure, molecules 12' of the phosphorescent material are arranged at random when the phosphorescent material is formed into a film as shown in FIG. 2B.

Accordingly, even when the phosphorescent material is excited to emit light, travelling directions of phosphorescence 16' are not equalized to a predetermined direction (normal line direction of thin film), i.e. transition moments of phosphorescence 16' are not equalized, and therefore the light emission luminance is relatively low. This is indicated by FIG. 2B where emitted phosphorescence 16' lies in various directions.

Depending on a relationship with the type of the central metal, the phosphorescent material of the present invention can stably emit phosphorescence having a high luminance by having a 2-phenylpyridine ligand in a molecule as represented by the general formula (1).

Further, the phosphorescent material has a relatively rigid β-diketone-type ligand (acetylacetonate ligand) in a molecule, whereby the metal complex can be stabilized, and the horizontal orientation when a film is formed can be further improved.

### (2) End substituents

When the phosphorescent material is constructed, R¹ and R² as end substituents may be bonded not only at a para-position but also at an ortho- or meta-position of an aryl ring to be substituted, and each is a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group.

That is, for example, when R¹ and R² each is a hydrogen atom, the heat resistance and stability of the phosphorescent material can be improved.

When R¹ and R² each is a predetermined alkyl group, for example a methyl group, an ethyl group, a n-propyl group, an iso-propyl group, a n-butyl group, a ter-butyl group (tertiary butyl group), an octyl group or the like, synthesis of the phosphorescent material becomes relatively easy, and can be stably produced. Particularly when R¹ and R² each is a tertiary butyl group, not only synthesis becomes easy, but also association of molecules of the phosphorescent material can be effectively prevented because the end substituents are bulky, and hence a light emission property with a higher luminance can

be achieved in the organic EL element.

When R¹ and R² each is a predetermined aryl group, for example a phenyl group, a biphenyl group, a ter-phenyl group or the like, the conjugated structure becomes more rigid, so that the horizontal orientation and stability of molecules of the phosphorescent material can be further improved.

When R¹ and R² each is a halogen atom or a boryl group, light emission quantum efficiency in the phosphorescent material can be further improved. It has been found that particularly when R¹ and R² each is a dimethylboryl group, a diphenyl boryl group or a ditolylboryl group among boryl groups, high light emission quantum efficiency is achieved.

Further, when R¹ and R² each is a predetermined amino group, the heat resistance and preservation stability of the phosphorescent material can be improved. It has been found that particularly when R¹ and R² each is a carbazole group among the predetermined amino groups, excellent stability is achieved.

### (3) Other substituents

When the phosphorescent material is constructed, groups a to l and o to s as substituents of the aryl ring each is preferably a hydrogen atom, an alkyl group having 1 to 20 carbon atoms, a substituted alkyl group having 1 to 20 carbon atoms, an aryl group having 6 to 20 carbon atoms, a substituted aryl group having 6 to 20 carbon atoms, or a halogen atom.

This is because predetermined phosphorescence is efficiently obtained by having these substituents.

Specific examples of the alkyl group having 1 to 20 carbon atoms include a methyl group, an ethyl group, a propyl group, an isopropyl group, a butyl group, a tertiary butyl group, a pentyl group, a hexyl group, an octyl group and the like.

Specific examples of the substituted alkyl group having 1 to 20 carbon atoms include a methyl group, an ethyl group, a propyl group, an isopropyl group, a butyl group, a tertiary butyl group, a pentyl group, a hexyl group, an octyl group and the like, which are partially substituted with a halogen atom.

Specific examples of the aryl group having 6 to 20 carbon atoms include a phenyl group, a benzyl group, a tolyl group, an o-xylyl group, a naphthyl group, a biphenyl group and the like.

Specific examples of the substituted aryl group having 6 to 20 carbon atoms include a phenyl group, a benzyl group, a tolyl group, an o-xylyl group, a naphthyl group, a biphenyl group and the like, which are partially substituted with a halogen atom.

Specific examples of the halogen atom include chlorine, bromine, fluorine and the like.

However, more preferably groups a to l and o to s as substituents of the aryl ring each is a hydrogen atom or a methyl group because the structure of the phosphorescent material becomes simple, and the phosphorescent material can be further stably and efficiently produced.

### (4) Central metal

When the phosphorescent material is constructed, the type of the central metal M is platinum (Pt) iridium (Ir), nickel (Ni), copper (Cu) or gold (Au).

This is because predetermined phosphorescence is efficiently obtained by forming a structure having the central metal described above.

However, more preferably the central metal is platinum (Pt) because phosphorescence can be emitted further stably with a higher luminance.

### (5) Repetition number

Although the repetition number can be determined in consideration of the linear conjugated structure, the repetition numbers m and n of the aryl ring each is an integer of 0 to 4 and m + n is an integer of 1 or greater.

This is because by having the repetition number described above, not only synthesis but also film formation by vapor deposition becomes easy, and phosphorescence can be stably emitted with a higher luminance.

Therefore, the repetition number is more preferably an integer of 1 to 3, further preferably an integer of 2 to 3.

### 2. Specific examples

Preferred examples of the phosphorescent material represented by the general formula (1) are as shown in the following formulae (2) to (10) and (12) to (14). Particularly, phosphorescent materials represented by the following formulae (2) to (10) are more preferable because when a light emitting element is constructed, phosphorescent having high internal quantum efficiency and a high luminance is stably obtained, and also good heat resistance and good dispersibility in a host material are achieved.

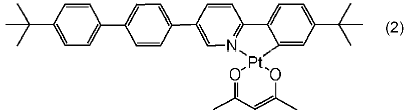

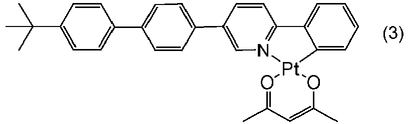

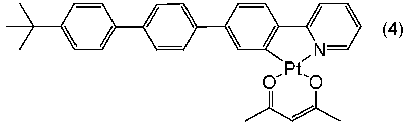

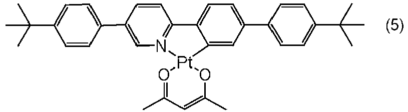

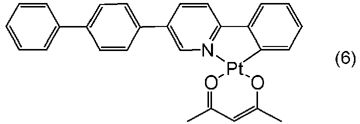

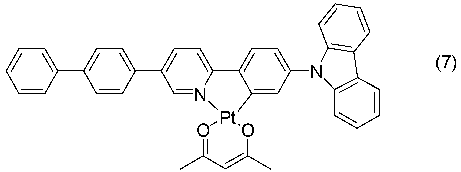

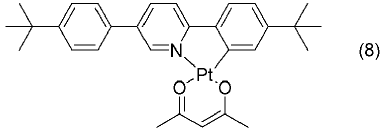

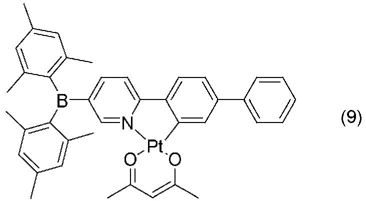

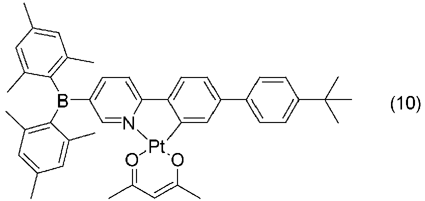

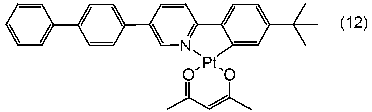

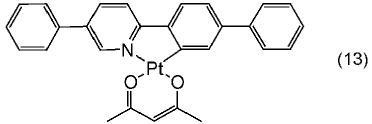

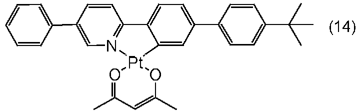

### 3. Stokes shift

When the phosphorescent material is constructed, the Stokes shift is set to preferably a value of 100 nm or more.

This is because by limiting the Stokes shift to the above-described range, phosphorescence having a further high luminance is efficiently obtained when the phosphorescent material is excited to emit light.

If the value of the Stokes shift becomes excessively large, the types of usable phosphorescent materials may be excessively limited.

Therefore, the Stokes shift is set to more preferably a value ranging from 120 to 180 nm, further preferably a value ranging from 125 to 150 nm.

The Stokes shift of the phosphorescent material is a phenomenon in which the line and band of a light emission spectrum are shifted to a longer wavelength side with respect to an absorption peak on the longest wavelength side, and the Stokes shift can be construed as a difference between the wavelength of excitation light and the wavelength of phosphorescence.

Thus, more specifically, the Stokes shift can be calculated by forming a thin film containing a predetermined amount of the phosphorescent material and measuring, for the thin film, an absorption peak on the longest wavelength side using an ultraviolet visible spectrophotometer UV-2550 (manufactured by Shimadzu Corporation), measuring an absorption peak of phosphorescent emission using a spectrophotofluorometer FP-6500-A-ST (manufactured by JASCO Corporation), and taking a difference between both the absorption peaks.

### 4. Weight-average molecular weight

The weight-average molecular weight of the phosphorescent material is set to preferably a value ranging from 400 to 1000.

This is because if the value of the weight-average molecular weight is less than 400, heat resistance and durability may be significantly deteriorated. On the other hand, if the value of the weight-average molecular weight is more than 1000, uniform dispersion in the host material may be difficult.

Thus, the weight-average molecular weight of the phosphorescent material is set to more preferably a value ranging from 410 to 800, further preferably a value ranging from 420 to 600.

The weight-average molecular weight can be measured by, for example, a gel permeation chromatography (GPC) by polystyrene conversion.

### 5. Optical properties

### (1) Polarizability

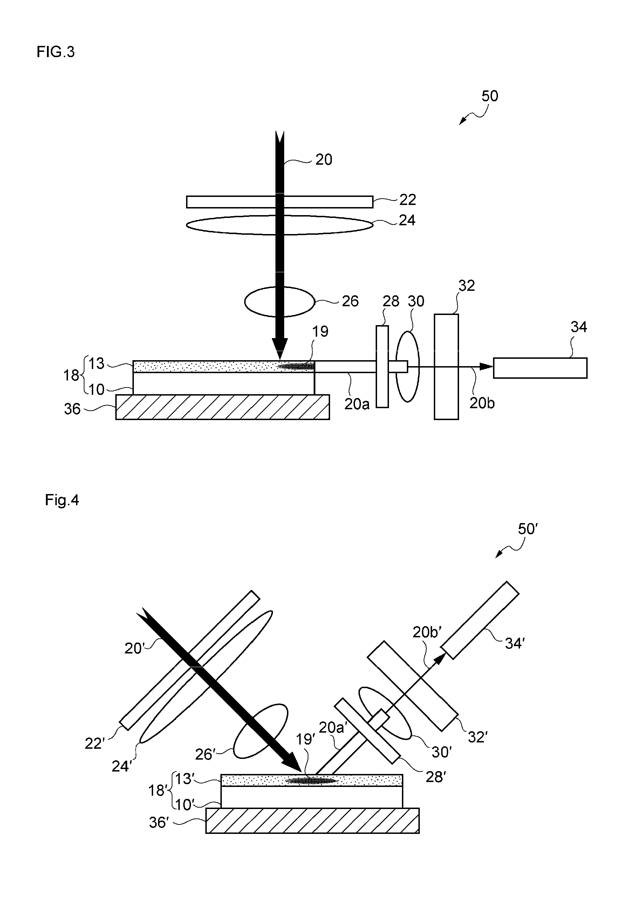

The polarizability of a predetermined phosphorescent material can be measured as one of optical properties using a measuring device 50 shown in FIG. 3.

That is, a predetermined thin film 13 containing the phosphorescent material is irradiated with laser light 20 in the direction of 90°, i.e. the normal line direction, to excite the phosphorescent material. A region 19 which emits light as a result of excitation of the phosphorescent material is shown in a part of the predetermined thin film 13 in FIG. 3.

Phosphorescence 20a is extracted from the end part of the predetermined thin film 13, and the phosphorescence 20a is caused to pass while the angle (0°, 30°, 60° and 90°) of a rotary polarizing plate 32 is changed, whereby an optical component 20b, the light polarization direction of which is consistent, can be extracted.

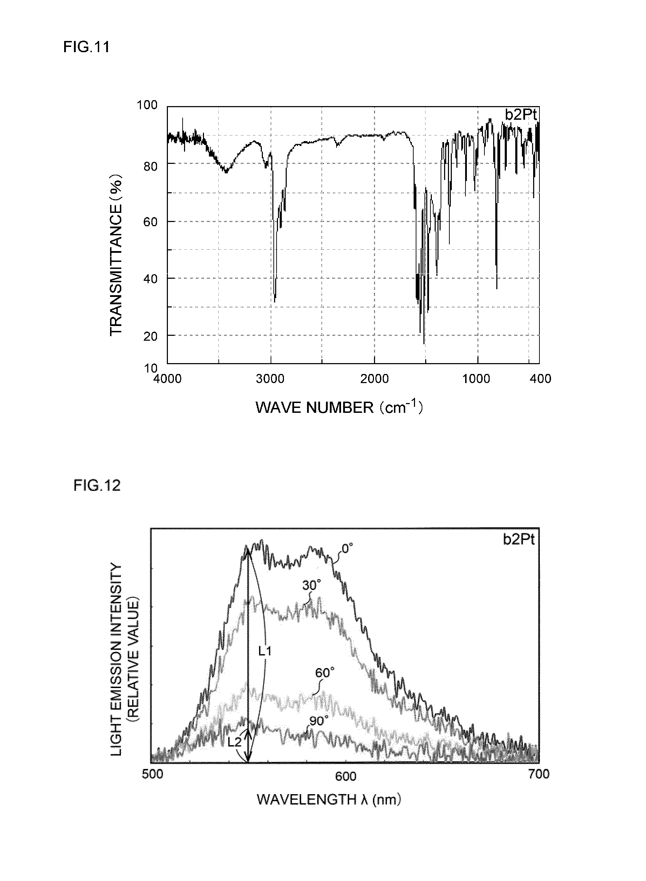

Therefore, as shown in FIG. 12 and the like, it may be preferred that the resulting phosphorescence 20b is composed of a mixture of optical components, the light emission luminance of which varies according to the angle of the rotary polarizing plate 32, i.e. the phosphorescent material has polarizability.

This is because when laser light is applied in the normal line direction to excite the phosphorescent material, only phosphorescence having a higher luminance can be extracted and used, although being a combination with a predetermined polarizing plate, owing to polarizability as described above.

Moreover, by appropriately changing the angle of the rotary polarizing plate 32, a plurality of rays of phosphorescence which is different in luminance can be sequentially extracted, and use can be made as a light source for decoration or a light source for data communication.

The measuring device 50 shown in FIG. 3 includes a sample stage 36 for placing a measurement sample 18 including a glass substrate 10 and a thin film sample 13, a ND filter 22 and a lens 24 for making only predetermined components of N₂ laser light 20 incident in the vertical direction, a diaphragm with a polarization eliminating filter 26 for narrowing an optical range, an optical filter 28 for cutting leakages of phosphorescence 20a emitted in the thin film sample 13 and incident laser light, a lens 30 for collecting light, a rotary polarizing filter 32, and a fiber scope 34 for detecting a polarization component 20b in phosphorescence.

In a phosphorescent emission spectrum obtained by the measuring device 50 shown in FIG. 3, a light emission ratio between angles 0° and 90° of the rotary polarizing plate 32 (e.g. wavelength: 550 nm) is called an aspect ratio, and from the aspect ratio, the polarizability of the phosphorescent material can be evaluated as shown in an example 1.

More specifically, the aspect ratio is expressed as L1/L2 if a light emission luminance of L1 is obtained when the angle of the rotary polarizing plate is 0°, and a light emission luminance of L2 is obtained when the angle of the rotary polarizing plate is 90° as shown in FIG. 12. In this case, it is understood that the value of L1 is about 6 times as large as the value of L2, i.e. the aspect ratio is about 6.

Accordingly, it can be said that larger the aspect ratio, higher the polarizability of the phosphorescent material, and by controlling the value of the aspect ratio, the polarizability of the phosphorescent material can be evaluated.

### (2) Non-polarizability

The non-polarizability of the phosphorescent material can be measured using a measuring device 50' shown in FIG. 4. That is, a predetermined thin film 13' containing the phosphorescent material is irradiated with laser light 20' from the direction of 45° left oblique, phosphorescence 20a' is simultaneously extracted from the direction of 45° right oblique, and phosphorescence 20a' is caused to pass while the angle (0°, 30°, 60° and 90°) of a rotary polarizing plate 32' is changed, whereby a non-polarizability component 20b' can be extracted.

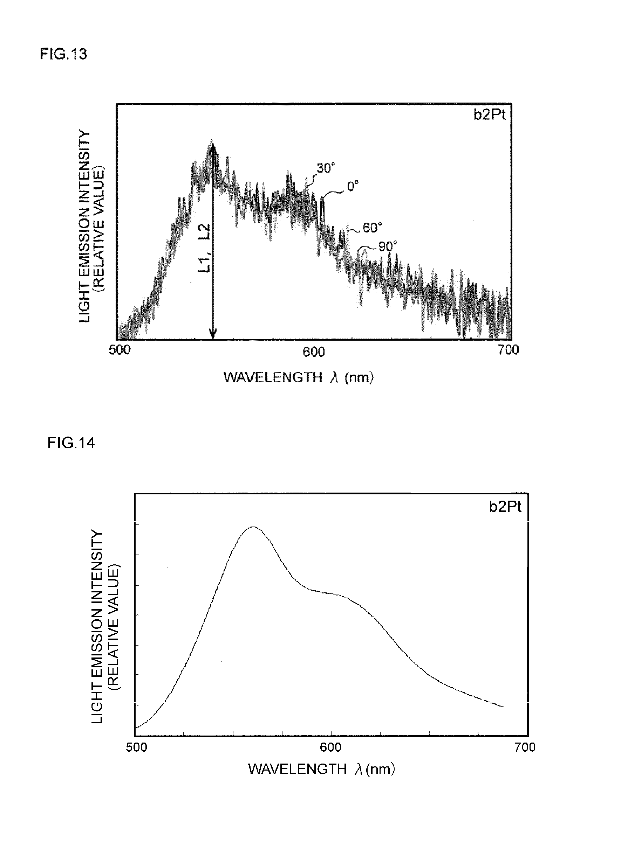

Therefore, as shown in FIG. 13 and the like, the phosphorescent material may have non-polarizability when light emission is observed at an angle where the light emission luminance does not vary according to the angle of the rotary polarizing plate 32', i.e. an angle in a non-horizontal direction with respect to a substrate, for example an angle of 45° with respect to the horizontal direction.

This is because even though the phosphorescent material has non-polarizability when excited by applying laser light in the non-normal line direction in this way, but since it has polarizability only when excited by applying laser light in the normal line direction, the resulting phosphorescence can be used as phosphorescence having a high luminance as described above.

The measuring device 50' shown in FIG. 4 is basically identical in configuration to the measuring device 50 shown in FIG. 3 except that only the incident angle of N₂ laser light is changed.

Thus, the measuring device 50' shown in FIG. 4 includes a sample stage 36' for placing a measurement sample 18' including a glass substrate 10' and a thin film sample 13', a ND filter 22' and a lens 24' for making only predetermined components of N₂ laser light 20' incident from the upper left angle direction of 45°, a diaphragm with a polarization eliminating filter 26' for narrowing an optical range, an optical filter 28' for cutting leakages of phosphorescence 20a' emitted in the thin film sample 13' and incident laser light, a lens 30' for collecting light, a rotary polarizing plate 32' , and a fiber scope 34' for detecting a polarization component 20b' in phosphorescence.

In a phosphorescent emission spectrum obtained by the measuring device 50' shown in FIG. 4, a light emission ratio between angles 0° and 90° of the rotary polarizing plate 32' (e.g. wavelength: 550 nm) is called an aspect ratio, and from the aspect ratio, the non-polarizability of the phosphorescent material can also be evaluated as shown in the example 1.

More specifically, the aspect ratio is expressed as L1/L2 if a light emission luminance of L1 is obtained when the angle of the rotary polarizing plate is 0°, and a light emission luminance of L2 is obtained when the angle of the rotary polarizing plate is 90° as shown in FIG. 13, but in this case, it is understood that the values of L1 and L2 are approximately the same, and the aspect ratio is close to 1.

Accordingly, it can be said that the phosphorescent material comes to have non-polarizability as the aspect ratio becomes closer to 1, and from the aspect ratio, the non-polarizability can be evaluated.

### (3) Horizontal orientation

As described above, the phosphorescent material of the first embodiment preferably shows a high aspect ratio and thus has polarizability when excited by applying laser light in the normal line direction, and may show a low aspect ratio and thus has non-polarizability when excited by applying laser light in the non-normal line direction.

When showing such a behavior, the phosphorescent material may be arranged in the horizontal direction as shown in FIG. 2A, i.e. show a good horizontal orientation.

On the other hand, if the aspect ratio is low, the phosphorescent material may be arranged not in the horizontal direction but at random as shown in FIG. 2B even when laser light is applied in the normal line direction.

In any case, the horizontal orientation of the phosphorescent material can be evaluated from the aspect ratio (L1/L2) of a phosphorescent emission spectrum or the like which is obtained by applying laser light in the normal line direction.

### 6. Internal quantum efficiency

The light emission quantum efficiency, i.e. internal quantum efficiency (φ), of the phosphorescent material when a light emitting element using the phosphorescent material is constructed is set to preferably a value of 30% or more.

This is because if the value of internal quantum efficiency is less than 30%, the light emission luminance of the resulting phosphorescent may be reduced, or extraction of polarization components may be difficult.

If the value of internal quantum efficiency of the phosphorescent material is more than 80%, the types of usable phosphorescent materials may be excessively limited.

Thus, internal quantum efficiency in the phosphorescent material is set to more preferably a value ranging from 40 to 75%, further preferably a value ranging from 50 to 70%.

Internal quantum efficiency in the phosphorescent material can be measured by a process described later in the example 1.

### 7. Aspect of use

The aspect of use of the phosphorescent material is not particularly limited, but examples thereof include a thin film, a tape shape, quadrangular prism shape, cylindrical shape, a conical shape, a spherical shape, an elliptic shape, a deformed shape and the like.

The process for forming a thin film or the like is not particularly limited, but for example, a vapor deposition process, a bar coating process, a knife coating process, a roll coating process, a blade coating process, a die coating process, a gravure coating process or the like can be used.

At this time, normally the thickness of the thin film is set to preferably a value ranging from 10 to 10000 nm, more preferably a value ranging from 50 to 5000 nm, further preferably a value ranging from 100 to 1000 nm.

Further, the phosphorescent material can be used alone, but is preferably used in mixture with various kinds of organic materials (polymer materials) and inorganic materials for securing good film formability and durability when a light emitting layer or the like is formed.

That is, when the phosphorescent material is used in mixture, the mixed quantity thereof is set to preferably a value ranging from 0.1 to 20% by weight based on the total amount of the light emitting layer or the like.

This is because the light emission luminance of the resulting phosphorescent may be significantly reduced if the value of the mixed quantity of the phosphorescent material is less than 0.1% by weight, while uniform dispersion may be difficult or durability may be deteriorated if the mixed quantity of the phosphorescent material is more than 20% by weight.

Therefore, the mixed quantity of the phosphorescent material is more preferably in a range of 1 to 12% by weight, further preferably in a range of 5 to 8% by weight based on the total amount of the light emitting layer or the like.

### [Second Embodiment]

The second embodiment of the present invention is a process for producing the phosphorescent material of the first embodiment, wherein the process includes the steps of: providing a binuclear complex represented by the following general formula (11); and acetylacetonating the binuclear complex.

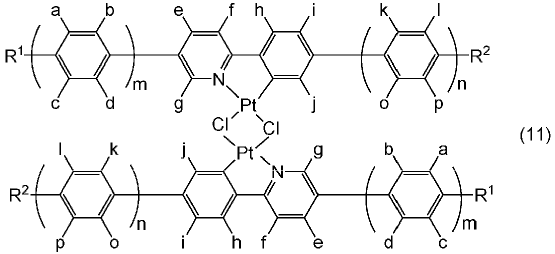

(In the general formula (11), a plurality of end substituents R¹ and R² is each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group, a plurality of substituents a to l and o to s is each independently a hydrogen atom, a substituted or unsubstituted alkyl group having 1 to 20 carbon atoms, a substituted or unsubstituted aryl group having 6 to 20 carbon atoms, or a halogen atom, a plurality of central metals M is each independently platinum (Pt), iridium (Ir), nickel (Ni), copper (Cu) or gold (Au), a plurality of repetition numbers m and n is each independently an integer of 0 to 4, and m + n is an integer of 1 or greater.)

The process for producing a phosphorescent material, which is the second embodiment of the present invention, will be specifically described below with reference to the drawings as appropriate.

### 1. Scheme for producing

The scheme for producing is a process for producing a phosphorescent material, which includes the steps of: providing a binuclear complex represented by the above general formula (11); and acetylacetonating the binuclear complex to form a phosphorescent material represented by the above general formula (1).

That is, when the binuclear complex represented by the general formula (11) is used as a starting material, two molecules of the phosphorescent material represented by the above general formula (1) are obtained simultaneously, and therefore there is the advantage that the phosphorescent material can be produced in a large quantity in a short time.

### 2. Providing step

The providing step is a step of synthesizing a binuclear complex including a pyridine structure represented by the general formula (11) before the acetylacetonating step.

That is, a straight-chain aryl compound including a pyridine structure can be synthesized with a compound having a pyridine ring by appropriately combining a Negishi coupling process including a coupling reaction of condensing an organic zinc compound and an organic halide under a palladium catalyst or the like and a Suzuki/Miyaura coupling process (SMC process) for obtaining an asymmetric biaryl by cross-coupling an organic boron compound and a halogenated aryl by means of actions of a palladium catalyst and a nucleophilic species of a base.

Then, the binuclear complex represented by the general formula (11) can be synthesized by using, for example, potassium tetrachloroplatinate and acetic acid, and the like based on the synthesized straight-chain aryl compound including a pyridine structure, and carrying out a heating treatment under conditions including a reaction temperature of 50 to 120°C and a reaction time of 1 to 100 hours in an inert gas (argon gas, nitrogen gas or the like).

### 3. Binuclear complex acetylacetonating step

The binuclear complex acetylacetonating step is a step of acetylacetonating the binuclear complex represented by the general formula (11), which is obtained through the providing step, using acetylacetone or the like, to prepare the phosphorescent material represented by the general formula (1).

### (1) Reaction temperature

First, the temperature at which a predetermined binuclear complex as a starting material is acetylacetonated, i.e. the reaction temperature, is set to preferably a value ranging from 50 to 150°C.

This is because if the value of the reaction temperature is less than 50°C, reaction efficiency is significantly deteriorated, or a by-product is liable to be easily generated.

On the other hand, if the value of the reaction temperature is more than 150°C, the reaction may excessively proceed, or the types of usable solvents may be excessively limited.

Thus, the reaction temperature is set to more preferably a value ranging from 60 to 120°C, further preferably a value ranging from 70 to 100°C.

### (2) Reaction time

The time during which a predetermined binuclear complex as a starting material is acetylacetonated, i.e. the reaction time, is set to preferably a value ranging from 1 to 100 hours.

This is because if the value of the reaction time is less than 1 hour, reaction efficiency is significantly deteriorated, or a by-product is liable to be easily generated.

On the other hand, if the value of the reaction time is more than 100 hours, producing efficiency is significantly deteriorated, or a by-product is liable to be easily generated.

Thus, the reaction time is set to more preferably a value ranging from 6 to 50 hours, further preferably a value ranging from 8 to 20 hours.

### (3) Catalyst

Potassium carbonate, sodium carbonate, calcium carbonate, potassium methoxide, sodium methoxide or the like is preferably used as a catalyst when a predetermined binuclear complex as a starting material is acetylacetonated.

This is because by using the above-mentioned catalyst, the predetermined binuclear complex can be efficiently acetylacetonated at a relatively low temperature.

### [Third Embodiment]

The third embodiment of the invention of the present application is a light emitting element which includes a light emitting layer or a plurality of organic thin film layers including the light emitting layer between a pair of electrodes including an anode and a cathode, wherein the light emitting layer contains a host material as a main component and the phosphorescent material of the first embodiment as a dopant material.

Principally the light emitting element (organic EL element, and the like), which is the third embodiment of the present invention, will be specifically described below with reference to the drawings as appropriate.

### 1. Basic Configuration

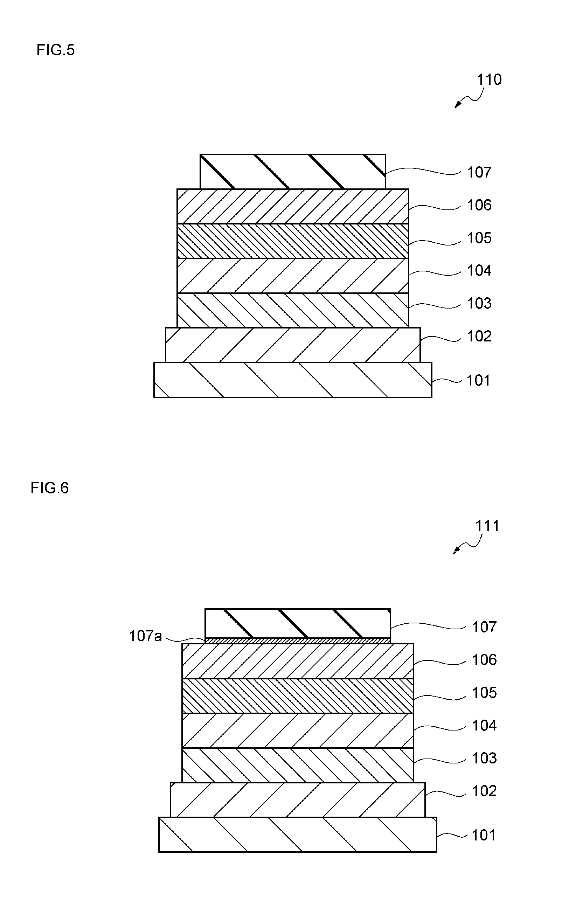

The light emitting element of the present invention, for example, typically an organic EL element 110 is formed by laminating, on a transparent substrate 101 such as glass, an anode 102 formed of a transparent electrically conductive material, a hole transport layer 103 formed of a predetermined organic compound, a light emitting layer 104 formed of a predetermined organic compound, a hole blocking layer 105 formed of a predetermined organic compound, an electron transport layer 106 formed of a predetermined organic compound and a cathode 107 formed of a metal material as shown in FIG. 5.

That is, with this multilayer structure as a basic configuration, the organic EL element 110 is constructed, and phosphorescence having a high luminance can be emitted by recombination of electrons and holes injected from the electrodes, respectively.

Also, as a structure of another organic EL element 111, an electron injection layer 107a as a thin film is laminated between the electron transport layer 106 and the cathode 107 as shown in FIG. 6.

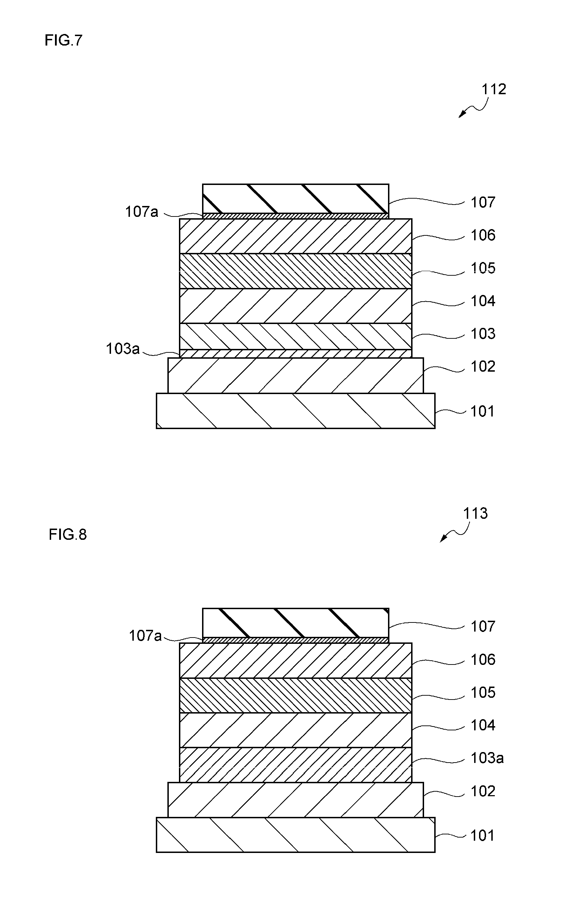

Further, it is also preferred that as a structure of another organic element 112, a hole injection layer 103a as a thin film is laminated between the anode 102 and the hole transport layer 103 as shown in FIG. 7.

Moreover, when the light emitting layer 104 is formed of a light emitting material having hole-transportability, the hole transport layer 103, the hole injection layer 103a and the like may be eliminated from the organic EL elements 110 to 112.

For example, the organic EL element 113 shown in FIG. 8 has a structure in which the substrate 101, the anode 102, the hole injection layer 103a, the light emitting layer 104, the hole blocking layer 105, the electron transport layer 106, the electron injection layer 107a and the cathode 107 are sequentially laminated from the bottom layer, and the hole transport layer 103 is not included.

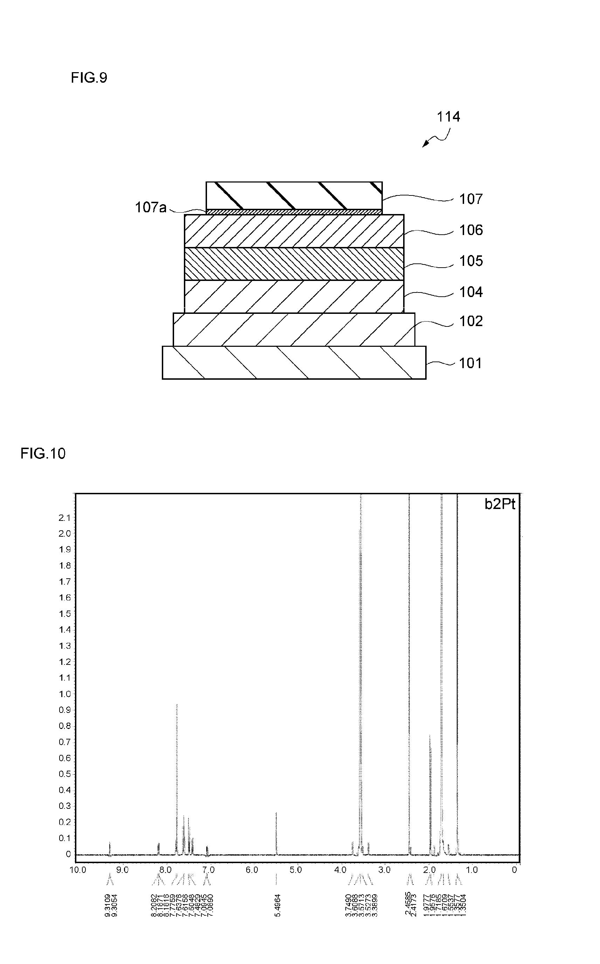

The organic EL element 114 shown in FIG. 9 has a structure in which the substrate 101, the anode 102, the light emitting layer 104, the hole blocking layer 105, the electron transport layer 106, the electron injection layer 107a and the cathode 107 are sequentially laminated from the bottom layer, and the hole transport layer 103 and the hole injection layer 103a are not included.

### 2. Light emitting layer

The light emitting layer contains, together with a host material, the phosphorescent material of the first embodiment as a dopant material for the host material.

Examples of the host material as a main component of the light emitting layer include a polyphenylene compound, a polyfluorene compound, a polythiophene compound, a polyphenylenevinylene compound, a polycarbazole compound, a polypyrrole compound, a polyacetylene compound, a polyaniline compound, a polyoxazole compound and the like alone or combinations of two or more thereof.

Particularly, the polycarbazole compound is preferable because it allows a high light emission luminance to be achieved, and is soluble in an organic solvent and easy to handle.

More specifically, mention is made of 4,4'-N,N'-dicarbazole-biphenyl (hereinafter, referred to as CBP in some cases) represented by the following formula (15), or 4,4',4"-tris(N-dicarbazolyl)triphenylamine represented by the following formula (16).

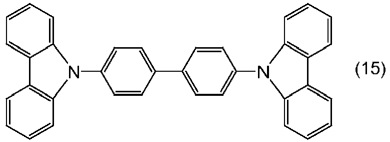

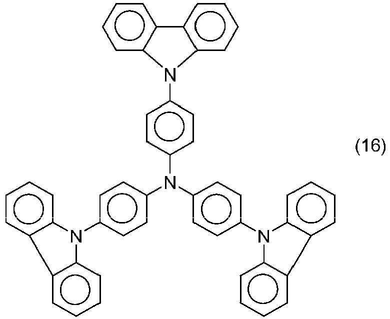

It is also preferred to add to the light emitting layer a material that supports electron transport. Examples of the electron transport supporting material include a triazole derivative, an oxazole derivative, a polycyclic compound, a heteropolycyclic compound such as bathocuproine, an oxadiazole derivative, a fluorenone derivative, a diphenyl quinone derivative, a thiopyran dioxide derivative, an anthraquinone dimethane derivative, an anthrone derivative, a carbodiimide derivative, a fluorenylidene methane derivative, a distyrylpyrazine derivative, an acid anhydride of an aromatic ring tetracarboxylic acid such as naphthalenetetracarboxylic acid or perylenetetracarboxylic acid, a phthalocyanine derivative, various kinds of metal complexes represented by a metal complex of a 8-quinolinol derivative or a metal phthalocyanine, a metal complex having a benzoxazole or benzothiazole as a ligand, an organic silane derivative, an iridium complex and the like alone or combinations of two or more thereof.

### 3. Anode

As the anode, a metal material or metal oxide material having a relatively large work function, more specifically a work function of 4 eV or more, is used.

As the metal material or the like, for example, at least one of indium tin oxide (ITO), indium zinc oxide (IZO), tin oxide (SnO₂), zinc oxide (ZnO) and the like is preferred.

Normally, the thickness of the anode is set to preferably a value ranging from 300 to 3000 angstroms.

### 4. Cathode

As the cathode, a metal material or metal oxide material having a relatively small work function, more specifically a work function of less than 4 eV, is used.

As the metal material or the like, for example, lithium, barium, aluminum, magnesium, indium, silver, an alloy thereof, or the like is preferred.

Normally, the thickness of the cathode is set to preferably a value ranging from 100 to 5000 angstroms.

When any one of the anode and cathode described above is transparent or translucent, predetermined phosphorescence can be extracted to outside.

### 5. Electron transport layer

Preferably at least the light emitting layer 104, anode 102 and cathode 107 described above, and the hole blocking layer 105 described later are included, and the electron transport layer 106 is provided at a predetermined position as shown in FIGS. 5 to 9.

Examples of the electron transport material that is mixed in the electron transport layer include a triazole derivative, an oxazole derivative, a polycyclic compound, a heteropolycyclic compound such as bathocuproine, an oxadiazole derivative, a fluorenone derivative, a diphenyl quinone derivative, a thiopyran dioxide derivative, an anthraquinone dimethane derivative, an anthrone derivative, a carbodiimide derivative, a fluorenylidene methane derivative, a distyrylpyrazine derivative, an acid anhydride of an aromatic ring tetracarboxylic acid such as naphthalenetetracarboxylic or perylenetetracarboxylic acid, a phthalocyanine derivative, various kinds of metal complexes represented by a metal complex of a 8-quinolinol derivative or a metal complex having a metal phthalocyanine, benzoxazole or benzothiazole as a ligand, an organic silane derivative, an iridium complex and the like alone or combinations of two or more thereof.

### 6. Hole blocking layer

Preferably the hole blocking layer 105 is provided as shown in FIGS. 5 to 9.

This is because by providing the hole blocking layer 105 as described above, light emission efficiency can be enhanced, and the life of the organic EL element can be increased.

Here, the hole blocking layer 105 can be provided using the aforementioned electron transport material, and is preferably a mixed layer formed by mixing two or more kinds of electron transport materials by co-deposition or the like.

Preferably the electron transport material contained in the hole blocking layer has an ionization potential larger than the ionization potential of the light emitting layer.

### 7. Others

Although not shown in the figure, it is preferred that the periphery of the display region of the organic RL element is sealed using an epoxy resin, an acrylic resin, or the like and using a predetermined member for eliminating influences of moisture to enhance durability.

It is preferred that a gap between the display region of the organic EL element and the predetermined member is filled with an inert gas such as nitrogen or argon, or an inert liquid such as fluorohydrocarbon or silicone oil.

On the other hand, it is also preferred that the gap is brought into vacuum or the gap is filled with a moisture absorbing compound for eliminating influences of moisture.

### Examples

The present invention will be described more in detail below by referring to examples.

### [Example 1]

### 1. Producing of phosphorescent material

### (1) Providing step 1

First, as shown in the reaction formula (1), 2-(4-tert-butylphenyl)-5-bromopyridine represented by the formula (20) was synthesized from 4-tert-butylbromobenzene represented by the formula (17).

That is, 4-tert-butylbromobenzene (0.83 ml, 1.01 g, 4.76 mmol) represented by the formula (17) was placed as a raw material compound in a container with a stirrer, 5 ml of tetrahydrofuran (THF) was then further added, and interior of the container was cooled to a temperature of -80°C under a nitrogen atmosphere.

Then, 3.1 ml (5.0 mmol) of a n-butyllithium/n-pentane solution at a concentration of 1.6 M was added, the temperature in the container was adjusted to -70°C, and the mixture was stirred for 40 minutes using the stirrer.

Then, 7 ml of a preliminarily prepared THF suspension of zinc chloride (zinc chloride: 0.64 g) was added into the container with a stirrer, the mixture was then heated to room temperature (20°C) for 50 minutes to obtain 4-tert-butyl-zinc chloride benzene represented by the formula (18).

Then, a solution obtained by dissolving 0.58 g of dichlorobis(triphenylphosphino) palladium in 5 ml of THF and 0.12 ml of a hydrogenated diisobutylaluminum/toluene solution at a concentration of 1.5 M were added into the container with a stirrer, and the mixture was stirred for 15 minutes.

Then, 2,5-dibromopyridine (1.10 g, 4.63 mmol) represented by the formula (19) was further added into the container with a stirrer, and the mixture was stirred for 20 hours.

Thereafter, the resulting solution was concentrated by an evaporator, 100 ml of acetic ether was added, and the mixture was washed with 100 ml of water.

Then, the washed product was washed with 100 ml of a saturated saline solution twice, then dried with magnesium sulfate, and concentrated by an evaporator.

Finally, the concentrate was purified by column chromatography (developing solvent: dichloromethane: n-hexane = 1 : 2) to obtain, as a white crystal, 2-(4-tert-butylphenyl)-5-bromopyridine (0.95 g) represented by the formula (20).

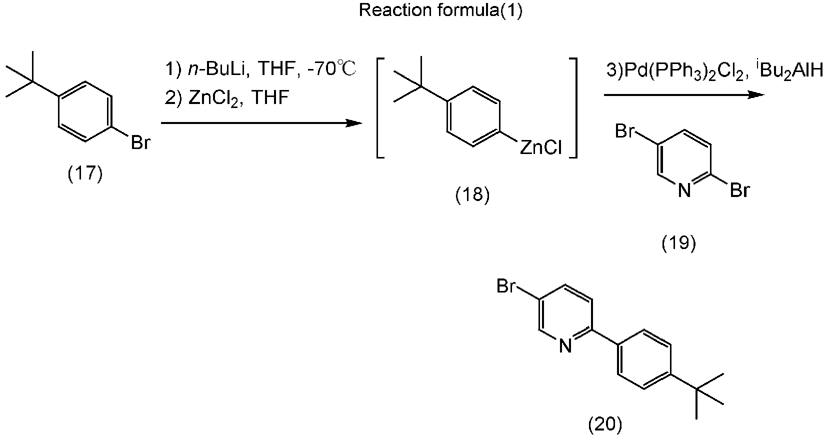

### (2) Providing step 2

On the other hand, as shown in the reaction formula (2), 2-{4-(4-tert-butylphenyl)phenyl}-4,4,5,5-tetramethyl-1,3,2-dioxaborolane represented by the formula (23) was synthesized from 4-(tert-butylphenyl)bromobenzene represented by the formula (21).

That is, 4-(tert-butylphenyl)bromobenzene (2.01 g, 6.92 mmol) represented by the formula (21) was placed in a container with a stirrer, 30 ml of THF was added, and interior of the container was cooled to a temperature of -80°C under a nitrogen atmosphere.

Then, 6.0 ml (9.7 mmol) of a 1.6 M
n-butyllithium/n-pentane solution was added, the temperature in the container was adjusted to -70°C, and the mixture was stirred for 30 minutes using the stirrer.

Then,
2-isopropoxy-4,4,5,5-tetramethyl-1,3,2-dioxaborolane (2.0 ml, 1.8 g, 9.7 mmol) represented by the formula (22) was added into the container with a stirrer, and the mixture was warmed to room temperature, and stirred for 20 hours.

Then, the resulting solution and 150 ml of water were mixed, the mixture was then extracted with 150 ml of acetic ether twice, and the resulting organic layer was washed with 150 ml of a saturated saline solution.

Then, the resulting organic layer was dried with magnesium sulfate, and concentrated by an evaporator.

Finally, the concentrate was recrystallized using dichloromethane/n-hexane to obtain, as a white crystal, 2-{4-(4-tert-butylphenyl)phenyl}-4,4,5,5-tetramethyl-1,3,2-dioxaborolane (1.47 g) represented by the formula (23).

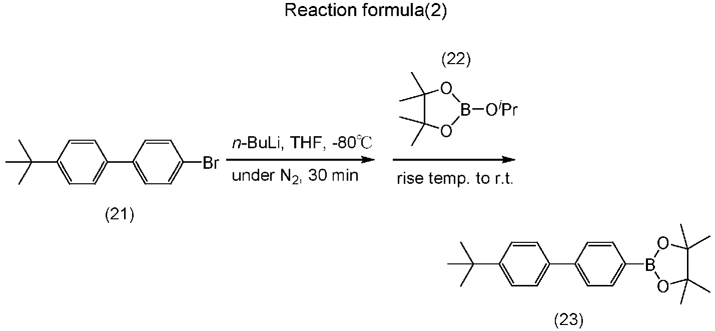

### (3) Providing step 3

As shown in the reaction formula (3), 2-(4-tert-butylphenyl)-5-{4-(4-tert-butylphenyl)phenyl}pyri -dine represented by the formula (24) was synthesized from 2-(4-tert-butylphenyl)-5-bromopyridine represented by the formula (20).

That is, 2-(4-tert-butylphenyl)-5-bromopyridine (0.29 g, 1.0 mmol) obtained in the providing step 1 and represented by the formula (20) was placed in a container with a stirrer, tetrakis(triphenylphosphine) palladium (0) (0.20 g, 0.18 mmol) and 20 ml of degassed THF were then added under a nitrogen atmosphere, and the mixture was stirred for 15 minutes. Then,
2-{4-(4-tert-butylphenyl)phenyl}-4,4,5,5-tetramethyl-1,3,2-dioxaborolane (0.337 g, 1 mmol) obtained in the providing step 2 and represented by the formula (23) was added into the container with a stirrer, 5 ml of a degassed aqueous sodium carbonate solution (concentration: 2 M) was then further added, and the mixture was heated and stirred at 60°C for 30 hours.

Then, the resulting solution was filtered, and the residue solid was recrystallized using dichloromethane/n-hexane to obtain, as a white crystal, 2-(4-tert-butylphenyl)-5-{4-(4-tert-butylphenyl)phenyl}pyri -dine (0.29 g, 0.69 mmol) represented by the formula (24).

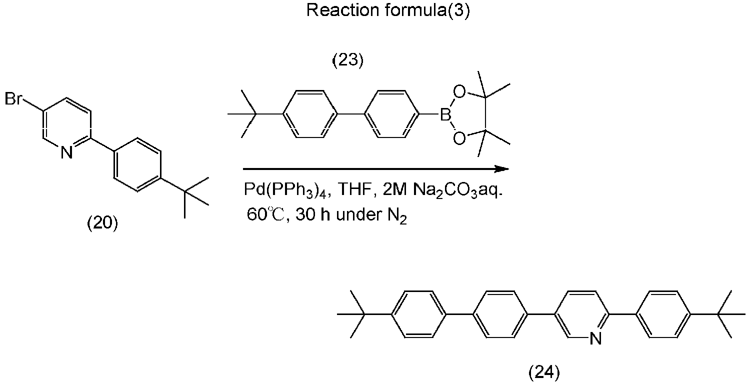

### (4) Providing step 4

As shown in the reaction formula (4), a binuclear complex represented by the formula (25), i.e. di-µ-chlorobis[1-[{4-(4-tert-butylphenyl)phenyl}-2-pyridyl-κN]-4-tert-butylphenyl-κC2] diplatinum (II) was synthesized from 2-(4-tert-butylphenyl)-5-{4-(4-tert-butylphenyl)phenyl}pyri -dine represented by the formula (24).

More specifically,
2-(4-tert-butylphenyl)-5-{4-(4-tert-butylphenyl)phenyl}pyri -dine (0.13 g, 0.31 mmol) obtained in the providing step 3 and represented by the formula (24) was placed in a container with a stirrer, 65 ml of acetic acid and an aqueous solution obtained by dissolving potassium tetrachloroplatinate (0.13 g, 0.31 mmol) in 2 ml of water were then added under an argon atmosphere, and the mixture was heated and stirred at 110°C for 20 hours.

Then, the resulting solution was filtered, and the residue solid was washed using water, methanol and dichloromethane to obtain, as a light green powder, a binuclear complex (0.15 g) represented by the formula (25).

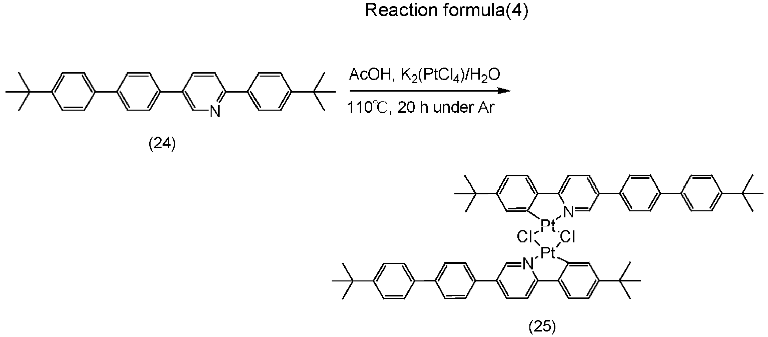

### (5) Acetylacetonating step

Then, as shown in the reaction formula (5), [acetylacetonate-κ02,κ04][1-[(4-(4-tert-butylphenyl)phenyl} -2-pyridyl-κN]-4-tert-butylphenyl-κC2] platinum (II) (abbreviated as b2Pt in some cases) represented by the formula (2) was obtained from the binuclear complex represented by the formula (25).

That is, the binuclear complex represented by the formula (25) was placed in a container with a stirrer, potassium carbonate (K₂CO₃, 0.16 g, 1.2 mmol) was then added in argon, and a solution obtained by dissolving acetylacetone (H(acac), 0.038 g, 0.38 mmol) in 10 ml of ethylcellosolve (ErOEtOH) was added. Thereafter, the mixture was heated and stirred at 100°C for 16 hours to obtain a predetermined reaction solution.

Finally, the resulting reaction solution was filtered, and the residue solid was recrystallized using THF/n-hexane to obtain, as a yellow powder, [acetylacetonate-κO2,κO4][1-[{4-(4-tert-butylphenyl)phenyl} -2-pyridyl-κN]-4-tert-butylphenyl-κC2] platinum (II) (0.11 g, 0.15 mmol) represented by the formula (2).

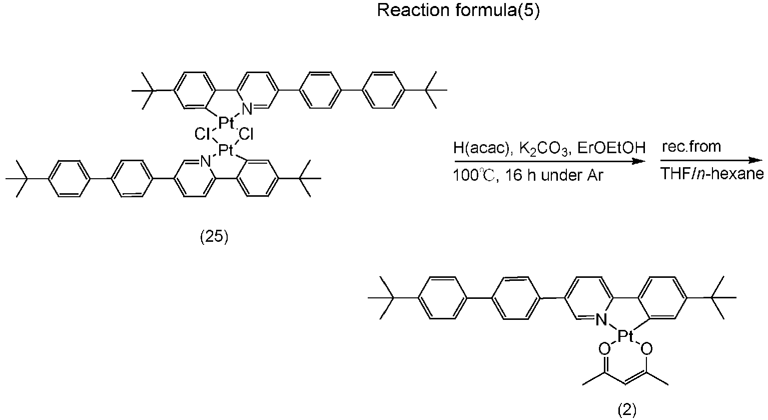

### 2. Formation of thin film of phosphorescent material

A thin film containing CBP as a host material and 6% by weight of the obtained phosphorescent material (b2Pt) as a dopant material was formed in a thickness of 100 nm on a glass substrate (made of borosilicate glass; size: 0.5 mm × 2.5 mm; thickness: 0.7 mm) by a co-deposition process.

Co-deposition conditions are as follows. Film formation device: Ultraprecise alignment mechanism-equipped vapor deposition device E-180-S (manufactured by ALS Technology Co., Ltd.)
Film formation speed: 1.6Å/sec.
Film formation pressure: 2.0 × 10⁻⁴Pa
Film formation time: 9.3 minutes

### 3. Evaluation of phosphorescent material

### (1) Evaluation of polarizability

Using a measuring device 50 shown in FIG. 3, laser light was applied in the normal line direction of the thin film of the phosphorescent material to excite the phosphorescent material, the polarizability (aspect ratio, i.e. light emission ratio between angles 0° and 90° of a rotary polarizing plate (wavelength: 550 nm) of phosphorescence thus obtained was measured, and evaluations were made according to the following criteria. The results thereof are shown in Table 1.

A phosphorescent emission spectrum obtained using the measuring device 50 shown in FIG. 3 is shown as FIG. 12. Very good: The aspect ratio is 4.0 or more.
Good: The aspect ratio is 3.0 or more and less than 4.0.
Fair: The aspect ratio is 2.0 or more and less than 3.0.
Bad: The aspect ratio is less than 2.0.

### (2) Evaluation of non-polarizability

Using a measuring device 50' shown in FIG. 4, laser light was applied from the direction of 45° left oblique to excite the phosphorescent material, the non-polarizability (aspect ratio, i.e. light emission ratio between angles 0° and 90° of a rotary polarizing plate (wavelength: 550 nm) )of phosphorescence) thus obtained was measured, and evaluations were made according to the following criteria. The results thereof are shown in Table 1.

A phosphorescent emission spectrum obtained using the measuring device 50' shown in FIG. 4 is shown as FIG. 13. Very good: The aspect ratio is less than 2.0.

Good: The aspect ratio is 2.0 or more and less than 3.0. Fair: The aspect ratio is 3.0 or more and less than 4.0. Bad: The aspect ratio is 4.0 or more.

### (3) Internal quantum efficiency

A predetermined thin film (thickness: 100 nm, concentration of phosphorescent material: 6% by weight, host material: compound represented by the formula (15)/CBP) containing the phosphorescent material was formed on a quartz substrate (7 × 15 mm), and internal quantum efficiency (light emission quantum efficiency) at a predetermined wavelength (337 nm) was measured using an absolute PL quantum yield measuring device C9920 (manufactured by Hamamatsu Photonics K.K.). The results obtained are shown in Table 1.

### (4) NMR

The NMR (nuclear magnetic resonance) of the phosphorescent material was measured using a nuclear magnetic resonance device JNM-EPC400 (manufactured by JEOL Ltd.) with the phosphorescent material dissolved in a heavy chloroform solvent. The NMR chart obtained is shown as FIG. 10.

### (5) FT-IR

A FT-IR chart of the phosphorescent material was measured by a KBr tablet process using a Fourier transform infrared spectrophotometer FT/IR-6100 (manufactured by JASCO Corporation). The FT-IR chart obtained is shown as FIG. 11.

### [Example 2]

In an example 2,
[acetylacetonate-κO2,κO4][1-[{4-(4-tert-butylphenyl)phenyl} -2-pyridyl-κN]-phenyl-κC2] platinum (II) (abbreviated as e1Pt in some cases) represented by the formula (3) was synthesized as a phosphorescent material, and evaluations were made in the same manner as in the example 1.

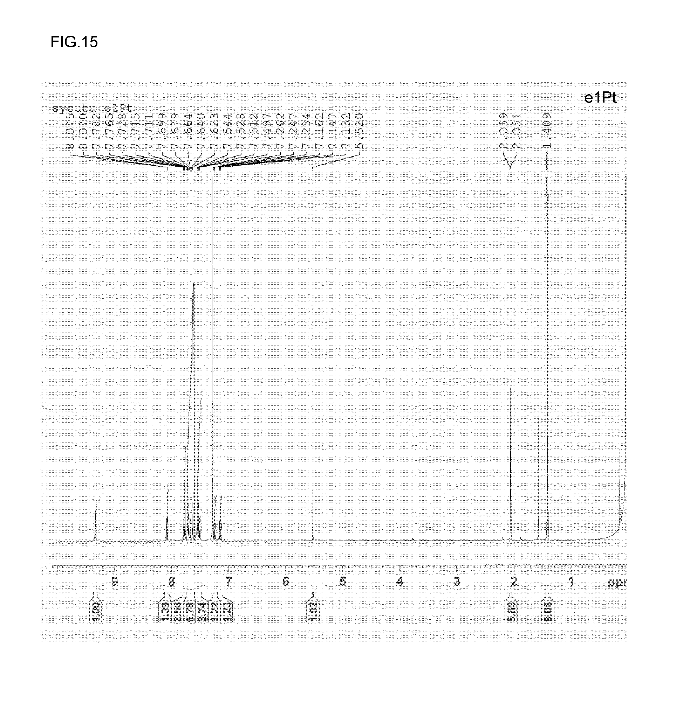

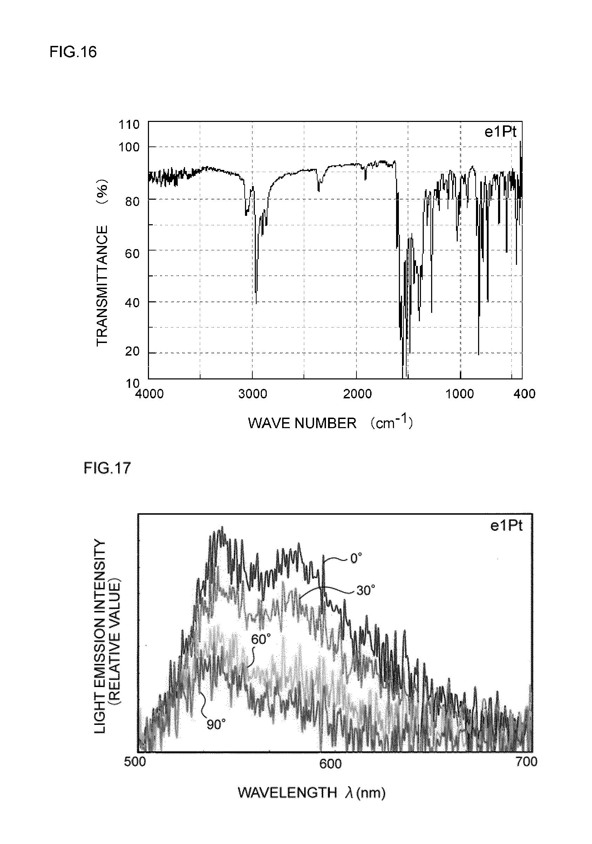

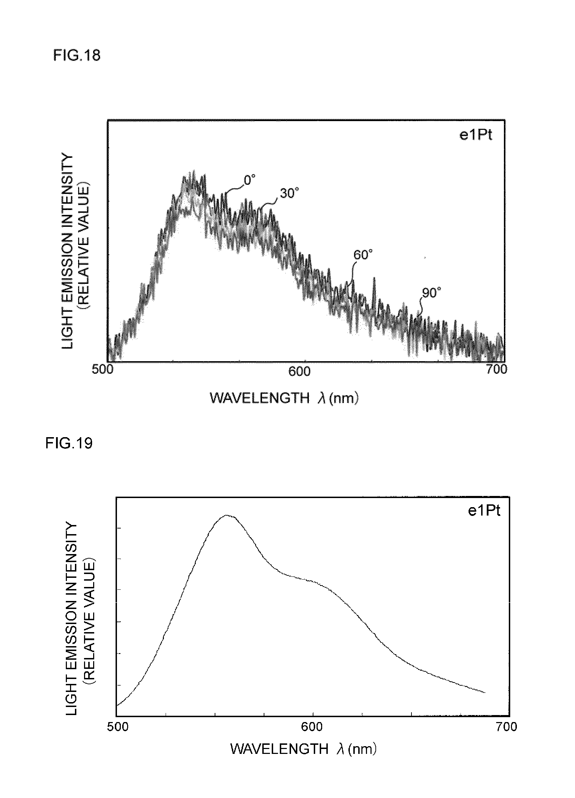

Accordingly, for the obtained e1Pt, a NMR chart is shown in FIG. 15, a FT-IR chart is shown in FIG. 16, a phosphorescent emission spectrum obtained by the measuring device shown in FIG. 3 is shown in FIG. 17, a phosphorescent emission spectrum obtained by the measuring device shown in FIG. 4 is shown in FIG. 18, and a phosphorescent emission spectrum obtained with the wavelength of excitation light set at 337 nm is shown in FIG. 19.

Further, in the example 2, an organic EL element including the obtained phosphorescent material in a light emitting layer was constructed, and evaluations were also made in terms of a material of the organic EL element.

### 1. Synthesis of phosphorescent material

### (1) Providing step 1

That is, as shown in the reaction formula (6), 2-phenyl-5-{4-(4-tert-butylphenyl)phenyl}pyridine represented by the formula (27) was synthesized from 5-bromo-2-phenylpyridine represented by the formula (26).

More specifically, 5-bromo-2-phenylpyridine (0.59 g, 2.5 mmol) represented by the formula (26) was placed in a container with a stirrer, tetrakis(triphenylphosphine) palladium (0) (0.59 g, 0.51 mmol) and 60 ml of degassed THF were then added under a nitrogen atmosphere, and the mixture was stirred for 15 minutes.

Then,
2-{4-(4-tert-butylphenyl)phenyl}-4,4,5,5-tetramethyl-1,3,2-dioxaborolane (0.84g, 2.5 mmol) represented by the formula (23) was added into the container with a stirrer, 15 ml of a degassed aqueous sodium carbonate solution at a concentration of 2 M was then further added, and the mixture was heated and stirred at 60°C for 20 hours.

Then, the resulting reaction solution was allowed to cool, 50 ml of water was added, the organic solvent was concentrated by an evaporator, and the resulting precipitate was filtered.

Finally, the resulting residue solid was recrystallized using dichloromethane/n-hexane to obtain, as a white crystal, 2-phenyl-5-{4-(4-tert-butylphenyl)phenyl}pyridine (0.74 g, 0.20 mmol) represented by the formula (27).

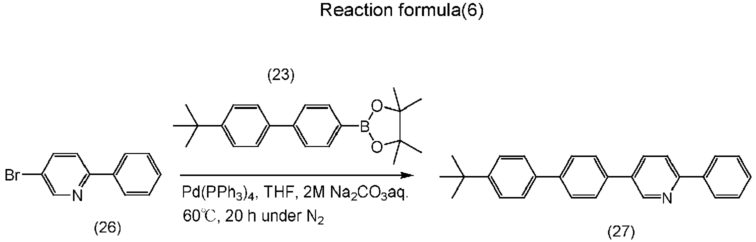

### (2) Providing step 2

Then, as shown in the reaction formula (7), a binuclear complex represented by the formula (28), i.e. di-µ-chlorobis[1-{4-(4-tert-butylphenyl)phenyl]-2-pyridyl-κ -N]phenyl-κC2] diplatinum (II) was synthesized from 2-phenyl-5-{4-(4-tert-butylphenyl)phenyl}pyridine represented by the formula (27).

More specifically,
2-phenyl-5-{4-(4-tert-butylphenyl)phenyl}pyridine (0.60 g, 1.7 mmol) represented by the formula (27) was placed in a container with a stirrer, 200 ml of acetic acid and an aqueous solution obtained by dissolving potassium tetrachloroplatinate (0.69 g, 1.7 mmol) in 20 ml of water were then added under an argon atmosphere, and the mixture was heated and stirred at 110°C for 16 hours.

Then, the resulting reaction solution was filtered, and the residue solid was washed with water, methanol and dichloromethane to obtain, as a yellow powder, a binuclear complex (0.64 g) represented by the formula (28).

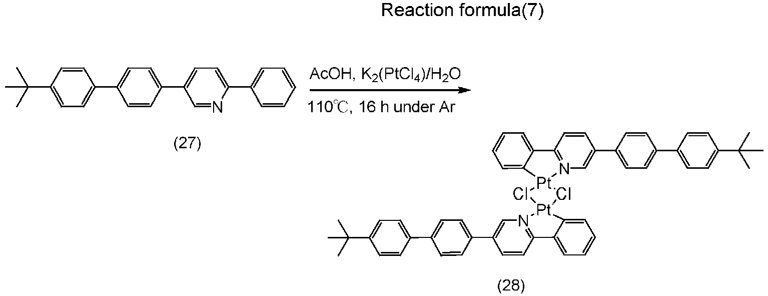

### (3) Acetylacetonating step

Then, as shown in the reaction formula (8), the binuclear complex represented by the formula (28) was subjected to an acetylacetonating step to synthesize
[acetylacetonate-κO2,κO4][1-[{4-(4-tert-butylphenyl)phenyl} -2-pyridyl-κN]-phenyl-κC2] platinum (II) represented by the formula (3).

More specifically, the binuclear complex (0.64 g) represented by the formula (28) was placed in a container with a stirrer, and potassium carbonate (0.75 g, 5.4 mmol) was then added under an argon atmosphere.

Then, a solution formed by dissolving acetylacetone (0.20 g, 2.0 mmol) in 40 ml of ethylcellosolve was added, and the mixture was then heated and stirred at 100°C for 14 hours.

Finally, the resulting reaction solution was filtered, and the residue solid was recrystallized using THF/n-hexane to obtain, as a yellow powder,
[acetylacetonate-κO2,κO4][1-[{4-(4-tert-butylphenyl)phenyl} -2-pyridyl-κN]-phenyl-κC2] platinum (II) (0.54 g, 0.82 mmol) represented by the formula (3).

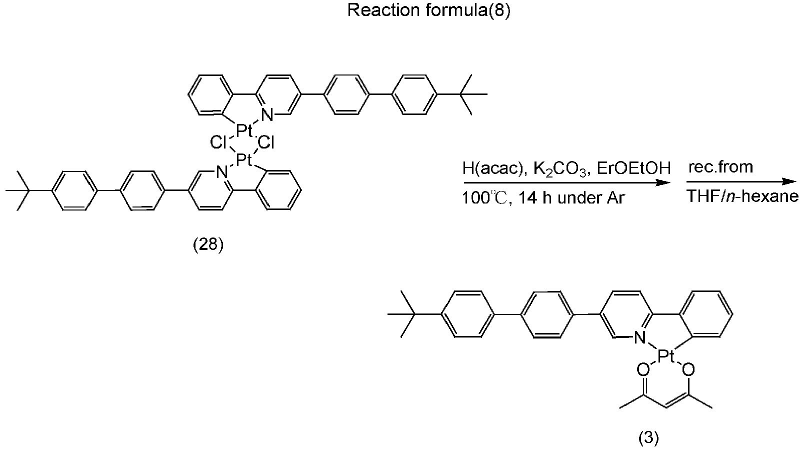

### 2. Producing and evaluation of organic EL element (1) Producing of organic EL element

A glass substrate (length of 12 mm, width of 12 mm and thickness of 1 mm) on which an indium tin oxide film having a thickness of 1000 Å was deposited was provided as an anode.

Thereon, a dimethyl naphthyl diamine-deposited layer (NPD, 40nm) was laminated as a hole transport layer, a N,N-dicarbazolyl-3,5-benzene-deposited layer (mCP, 10 nm) was laminated as an electron blocking layer, a mCP-deposited layer (20 nm) containing, at a concentration of 6% by weight, e1Pt represented by the above formula (3) was laminated as a light emitting layer, a 2,9-dimethyl-4,7-diphenyl-1,10-phenanthroline-deposited layer (BCP, 10 nm)was laminated as a hole blocking layer, a tris(8-hydroxyquinoline) aluminum-deposited layer (Alq₃, 40 nm) was laminated as an electron transport layer, a MgAg (100 nm)-deposited layer and an Ag (20 nm)-deposited layer were laminated as a cathode, and an electric power source was connected thereto to form an organic EL element.

### (2) Evaluation of organic EL element

A voltage (V) applied to the obtained organic EL element was changed, and a current density (mA/cm²) of a current passing through the interior of the organic EL element was measured using a digital volt meter.

Further, external quantum efficiency (%) as an index defined by (number of photons extracted from organic EL element / number of photons injected into organic EL element) × 100, which was calculated from the amount of light or the like in an EL light emission spectrum when the current density (mA/cm²) of a current passing through the interior of the organic EL element was changed, was calculated.

As a result, the external quantum efficiency was 15.8% (internal quantum efficiency by optical excitation of light emitting layer: 50%, light extraction efficiency from light emitting layer: 31.6%).

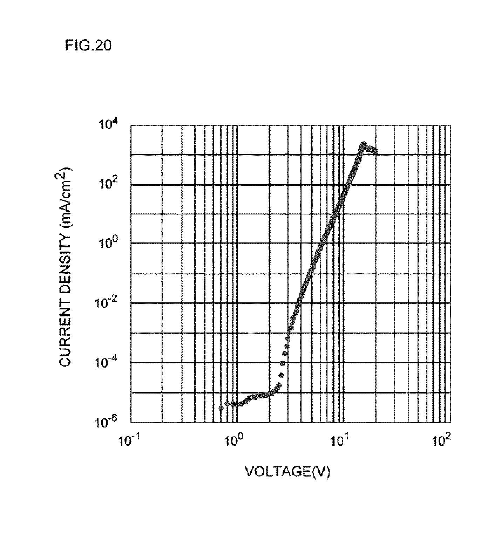

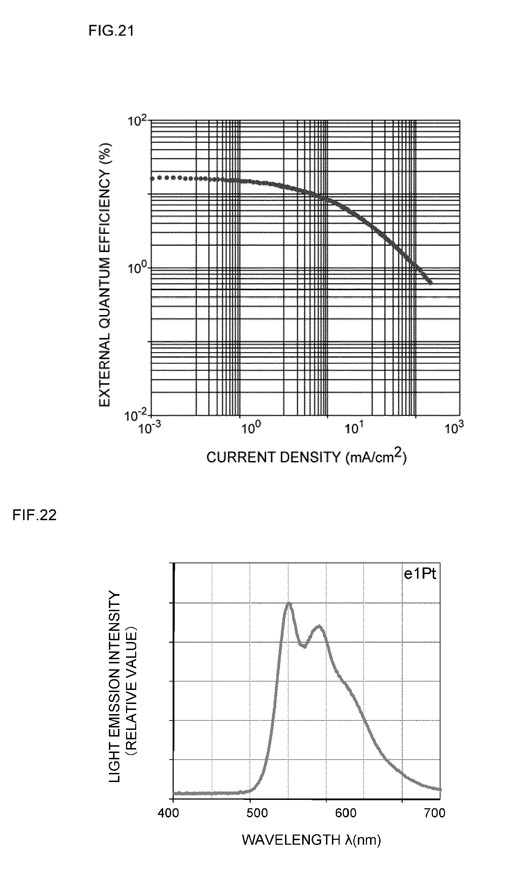

A relationship between the voltage (V) and the current density (mA/cm²) in the organic EL element is shown in FIG. 20, a relationship between the current density (mA/cm²) and the external quantum efficiency in the organic EL element is shown in FIG. 21, and a light emission spectrum (current value: 1 mA/cm²) in the organic EL element is shown in FIG. 22.

### [Example 3]

In an example 3,
[acetylacetonate-κO2,κO4] [1-(2-pyridyl-κN)-4-{4-(4-tert-but ylphenyl)phenyl}phenyl-κC2] platinum (II) (abbreviated as f1Pt in some cases) represented by the formula (4) was synthesized as a phosphorescent material, and evaluations were made in the same manner as in the example 1.

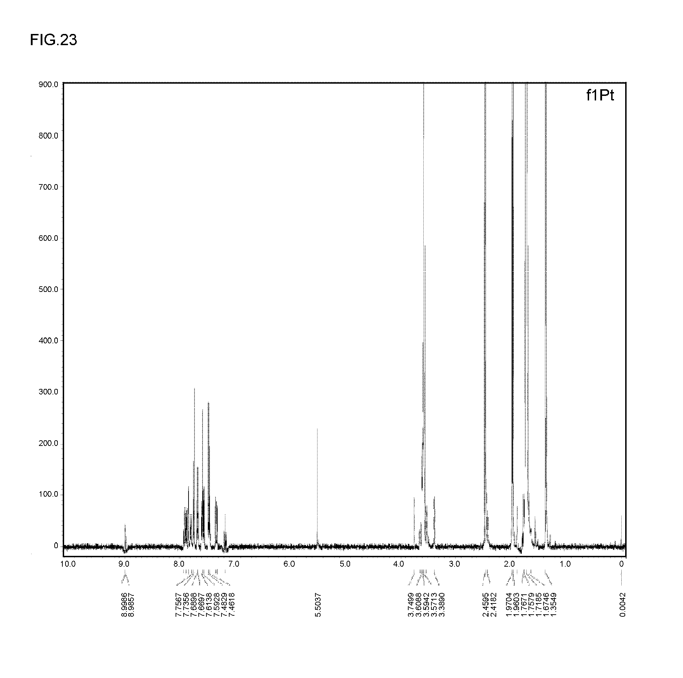

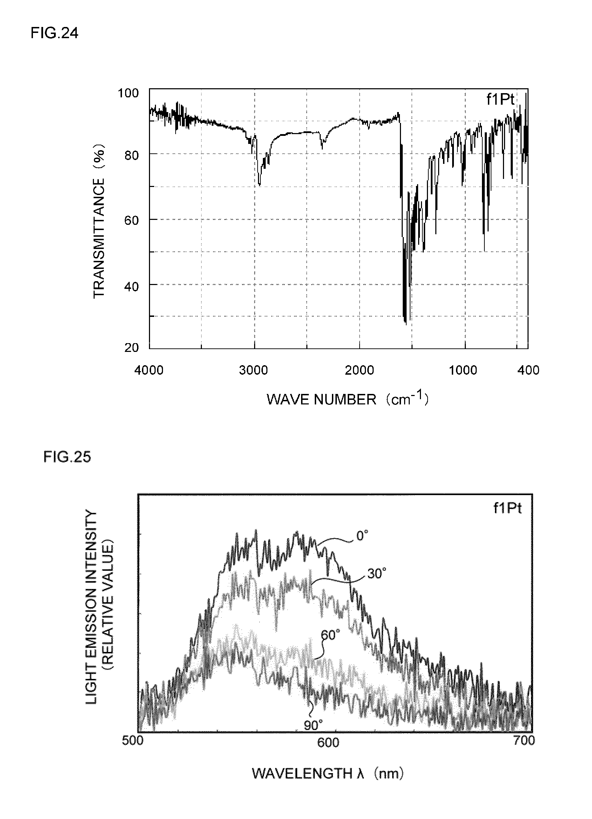

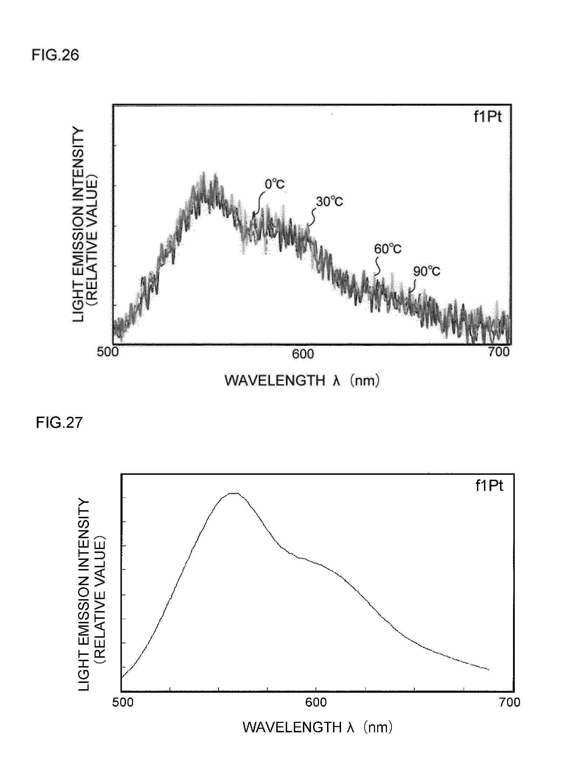

For the obtained f1Pt, a NMR chart is shown in FIG. 23, a FT-IR chart is shown in FIG. 24, a phosphorescent emission spectrum obtained by the measuring device shown in FIG. 3 is shown in FIG. 25, a phosphorescent emission spectrum obtained by the measuring device shown in FIG. 4 is shown in FIG. 26, and a phosphorescent emission spectrum obtained with the wavelength of excitation light set at 337 nm is shown in FIG. 27.

### (1) Providing step 1

That is, as shown in the reaction formula (9), 2-[4-{4-(4-tert-butylphenyl)phenyl]pyridine represented by the formula (31) was synthesized from 2-{4-(4-bromophenyl)phenyl}pyridine represented by the formula (29).

More specifically, 2-{4-(4-bromophenyl)phenyl}pyridine (0.31 g, 1.0 mmol)represented by the formula (29) was placed in a container with a stirrer, tetrakis(triphenylphosphine) palladium (0) (0.19 g, 0.16 mmol) and 20 ml of degassed THF were then further added under a nitrogen atmosphere, and the mixture was stirred for 15 minutes.

Then, to the resulting solution was added 2-(4-tert-butylphenyl)-4,4,5,5-tetramethyl-1,3,2-dioxaborol ane (0.26 g, 1.0 mmol) represented by the formula (30), 5 ml of a degassed aqueous sodium carbonate solution at a concentration of 2 M was further added, and the mixture was heated and stirred at 60°C for 20 hours.

Then, the resulting reaction solution was allowed to cool, and the resulting precipitate was filtered.

Then, the resulting residue solid was washed with water and a small amount of THF, and then recrystallized using dichloromethane/n-hexane to obtain, as a white crystal, 2-[4-{4-(4-tert-butylphenyl)phenyl]phenyl}pyridine (0.16 g, 0.43 mmol) represented by the formula (31).

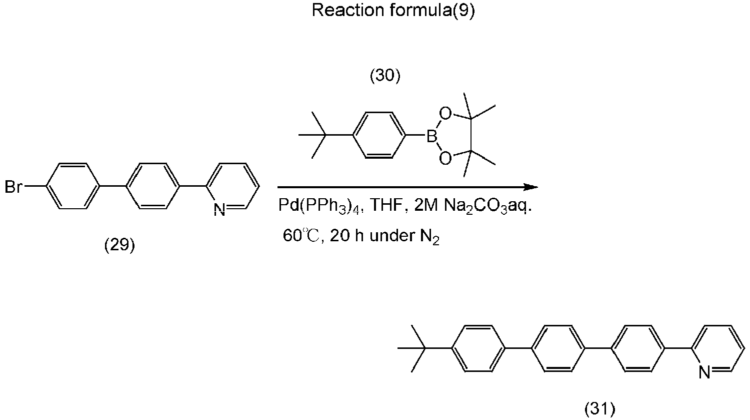

### (2) Providing step 2

Then, as shown in the reaction formula (10), a binuclear complex represented by the formula (32), i.e. di-µ-chlorobis[1-(2-pyridyl-κN)-4-{4-(4-tert-butylphenyl)ph -enyl]-κC2] diplatinum (II) was synthesized from 2-[4-{4-(4-tert-butylphenyl)phenyl]phenyl}pyridine represented by the formula (31).

More specifically, 2-[4-{4-(4-tert-butylphenyl)phenyl]phenyl}pyridine (0.088 g, 0.24 mmol) represented by the formula (31) was placed in a container with a stirrer, 50 ml of acetic acid and an aqueous solution obtained by dissolving potassium tetrachloroplatinate (0.10 g, 0.24 mmol) in 3 ml of water were then further added under an argon atmosphere, and the mixture was heated and stirred at 110°C for 16 hours.

Then, the resulting solution was filtered, and the residue solid was then washed with water, methanol and dichloromethane to obtain, as a yellow powder, a binuclear complex (0.13 g) represented by the formula (32).

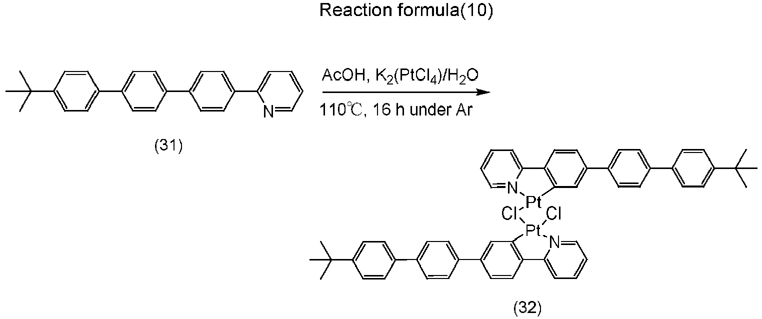

### (3) Acetylacetonating step

Then, as shown in the reaction formula (11), the binuclear complex represented by the formula (32) was subjected to an acetylacetonating step to synthesize [acetylacetonate-κO2,κO4] [1-(2-pyridyl-κN)-4-{4-(4-tert-but -ylphenyl)phenyl}phenyl-κC2] platinum (II) represented by the formula (4).

More specifically, the binuclear complex (0.13 g) represented by the formula (32) was placed in a container with a stirrer, potassium carbonate (0.15 g, 1.1 mmol) and a solution obtained by dissolving acetylacetone (0.055 g, 0.55 mmol) in 12 ml of ethylcellosolve were then added under an argon atmosphere, and the mixture was heated and stirred at 100°C for 22 hours.

Finally, the resulting reaction solution was filtered, and the resulting residue solid was recrystallized using THF/n-hexane to obtain, as a yellow powder, [acetylacetonate-κO2,κO4] [1-(2-pyridyl-κN)-4-{4-(4-tert-but -ylphenyl)phenyl}phenyl-κC2] platinum (II) (0.067 g, 0.10 mmol) represented by the formula (4).

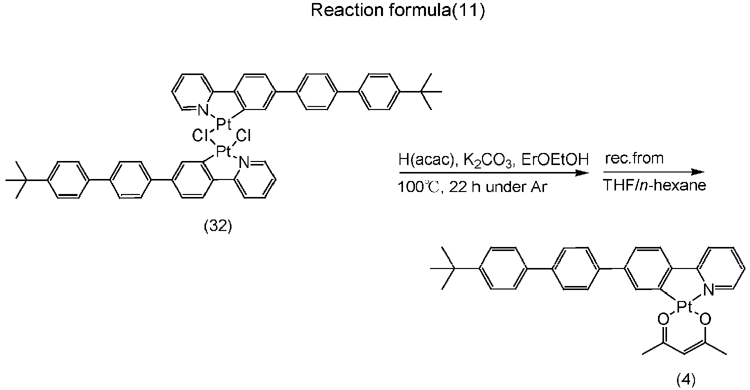

### [Example 4]

In an example 4,
[acetylacetonate-κO2,κO4] [1-{(4-tert-butylphenyl)-(2-pyridy -1-κN)-4-(4-tert-butylphenyl)phenyl-κC2] platinum (II) (abbreviated as c2Pt in some cases) represented by the formula (5) was synthesized as a phosphorescent material, and evaluations were made in the same manner as in the example 1.

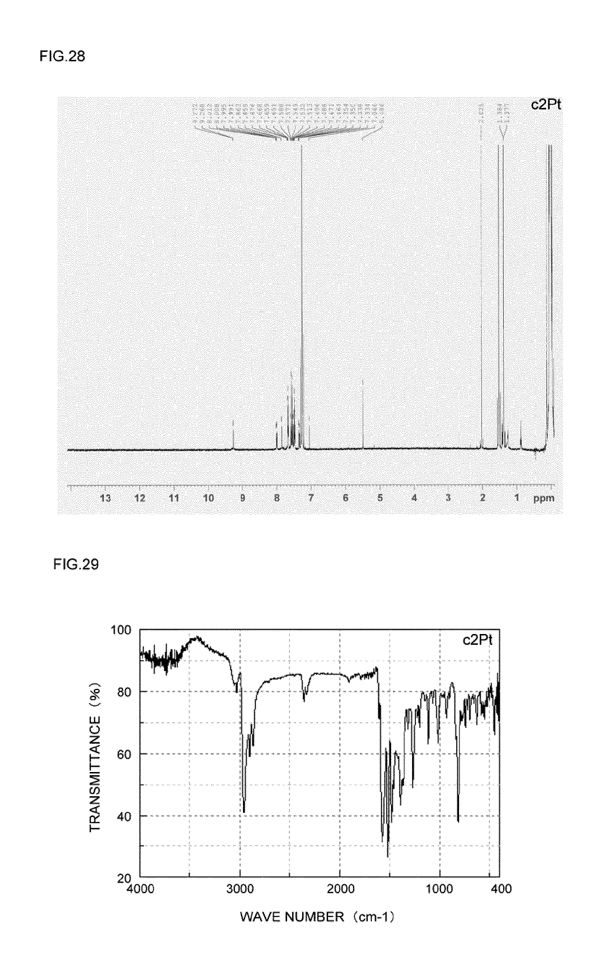

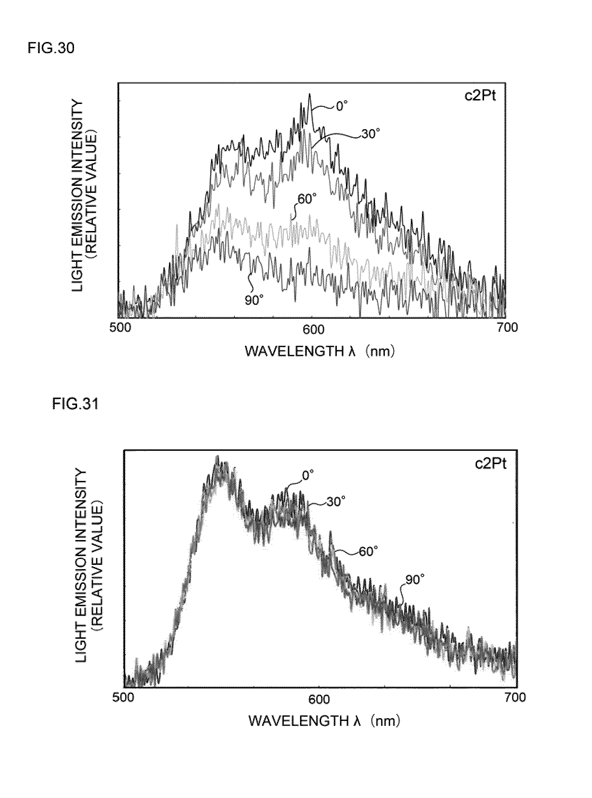

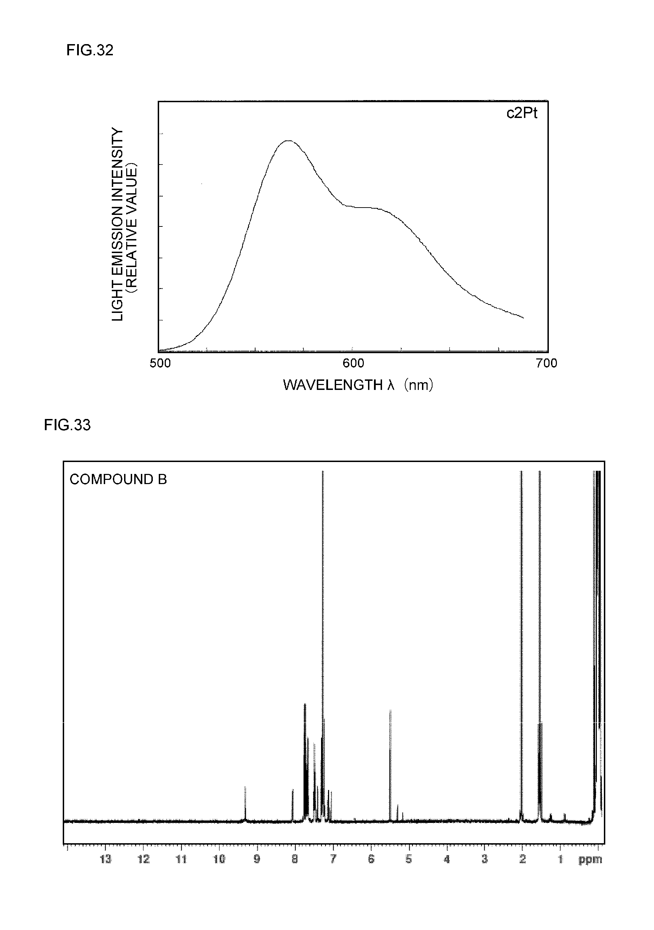

For the obtained c2Pt, a NMR chart is shown in FIG. 28, a FT-IR chart is shown in FIG. 29, a phosphorescent emission spectrum obtained by the measuring device shown in FIG. 3 is shown in FIG. 30, a phosphorescent emission spectrum obtained by the measuring device shown in FIG. 4 is shown in FIG. 31, and a phosphorescent emission spectrum obtained with the wavelength of excitation light set at 337 nm is shown in FIG. 32.

### (1) Providing step 1

That is, as shown in the reaction formula (12), 2-{4-(4-tert-butylphenyl)phenyl]-5-bromopyridine represented by the formula (34) was synthesized from 2,5-dibromopyridine represented by the formula (33).

More specifically, 2,5-dibromopyridine (0.95 g, 4.0 mmol) represented by the formula (33) was placed in a container with a stirrer, tetrakis(triphenylphosphine) palladium (0) (0.80 g, 0.69 mmol) and 80 ml of degassed THF were then added under a nitrogen atmosphere, and the mixture was stirred for 15 minutes.

Then,
2-{4-(4-tert-butylphenyl)phenyl}-4,4,5,5-tetramethyl-1,3,2-dioxaborolane (1.3 g, 4.0 mmol) represented by the formula (23) was added to the resulting solution, 20 ml of a degassed aqueous sodium carbonate solution at a concentration of 2 M was then further added, and the mixture was heated and stirred at 60°C for 24 hours.

Then, the resulting reaction solution was allowed to cool, 60 ml of 10% aqueous ammonia was then added, and the mixture was extracted with 60 ml of acetic ether twice.

Then, the resulting organic layer was washed with 80 ml of a saturated saline solution, then dried with magnesium sulfate, and concentrated by an evaporator.

Finally, the resulting concentrate was purified by column chromatography (developing solvent: dichloromethane: n-hexane: triethylamine = 20 : 20 : 1) to obtain, as a white crystal, 2-{4-(4-tert-butylphenyl)phenyl}-5-bromopyridine (0.54 g, 1.5 mmol) represented by the formula (34).

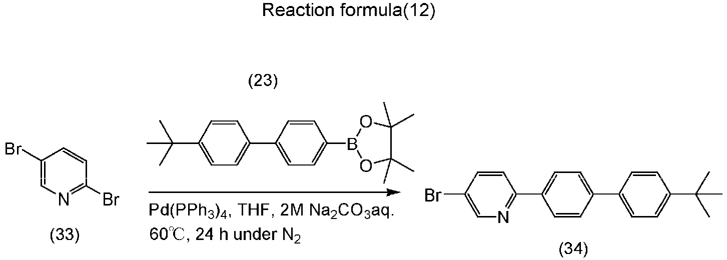

### (2) Providing step 2

Then, as shown in the reaction formula (13), 2-{4-(4-tert-butylphenyl)phenyl}-5-(4-tert-butylphenyl)pyri -dine represented by the formula (35) was synthesized from 2-{4-(4-tert-butylphenyl)phenyl}-5-bromopyridine represented by the formula (34).

More specifically,
2-{4-(4-tert-butylphenyl)phenyl}-5-bromopyridine (0.54 g, 1.5 mmol) represented by the formula (34) was placed in a container with a stirrer, tetrakis(triphenylphosphine) palladium (0) (0.35 g, 0.30 mmol) and 30 ml of degassed THF were then added under a nitrogen atmosphere, and the mixture was stirred for 15 minutes.

Then, to the resulting solution was added 2-(4-tert-butylphenyl)-4,4,5,5-tetramethyl-1,3,2-dioxaborol -ane (0.39 g, 1.5 mmol) represented by the formula (30), 7.5 ml of a degassed 2 M aqueous sodium carbonate solution was further added, and the mixture was heated and stirred at 60°C for 3 days.

Then, the resulting reaction solution was allowed to cool, 50 ml of water was then added, the mixture was extracted with 50 ml of dichloromethane twice, further the organic layer was washed with 100 ml of a saturated saline solution, and the solution was dried with magnesium sulfate, and concentrated by an evaporator.

Finally, the resulting concentrate was purified by column chromatography (developing solvent: dichloromethane:
n-hexane: triethylamine = 20 : 20 : 1) to obtain, as a yellowish white crystal,
2-{4-(4-tert-butylphenyl)phenyl}-5-(4-tert-butylphenyl)pyri -dine (0.22 g, 0.52 mmol) represented by the formula (35).

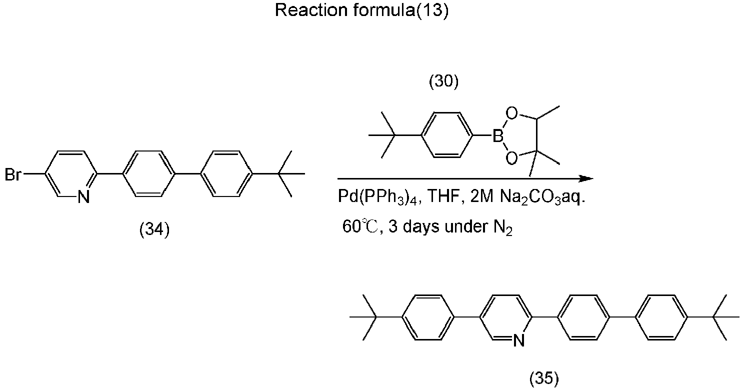

### (3) Providing step 3

Then, as shown in the reaction formula (14), a binuclear complex represented by the formula (36), i.e. di-µ-chlorobis[1-{(4-tert-butylphenyl)-2-pyridyl-κN]-4-(4-t -ert-butylphenyl)phenyl-κC2] diplatinum (II) was synthesized from 2-{4-(4-tert-butylphenyl)phenyl}-5-(4-tert-butylphenyl)pyri -dine represented by the formula (35).

More specifically,
2-{4-(4-tert-butylphenyl)phenyl}-5-(4-tert-butylphenyl)pyri -dine (0.18 g, 0.43 mmol) represented by the formula (35) was placed in a container with a stirrer, 100 ml of acetic acid and an aqueous solution obtained by dissolving potassium tetrachloroplatinate (0.18 g, 0.43 mmol) in 5 ml of water were then further added under an argon atmosphere, and the mixture was heated and stirred at 110°C for 18 hours.

Then, the resulting reaction solution was filtered, and the residue solid was washed with water, methanol and dichloromethane to obtain, as a dark green powder, a binuclear complex (0.16 g) represented by the formula (36).

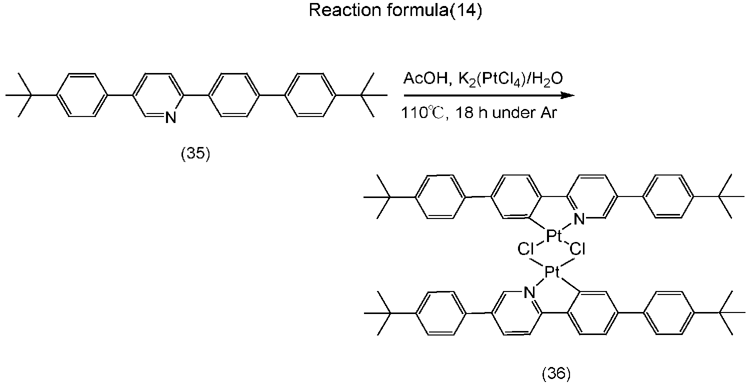

### (4) Acetylacetonating step

Then, as shown in the reaction formula (15), to the binuclear complex (0.16 g) represented by the formula (36), under an argon atmosphere, was added potassium carbonate (0.17 g, 1.2 mmol) and was added a solution obtained by dissolving acetylacetone (0.060 g, 0.60 mmol) in 10 ml of ethylcellosolve, and the mixture was heated and stirred at 100°C for 18 hours.

Finally, the resulting reaction solution was filtered, and the resulting residue solid was recrystallized using THF/n-hexane to obtain, as a faint orange powder, [acetylacetonate-κO2,κO4] [1-{(4-tert-butylphenyl)-(2-pyridy -1-κN)-4-(4-tert-butylphenyl)phenyl-κC2] platinum (II) (0.083 g, 0.12 mmol) represented by the formula (5).

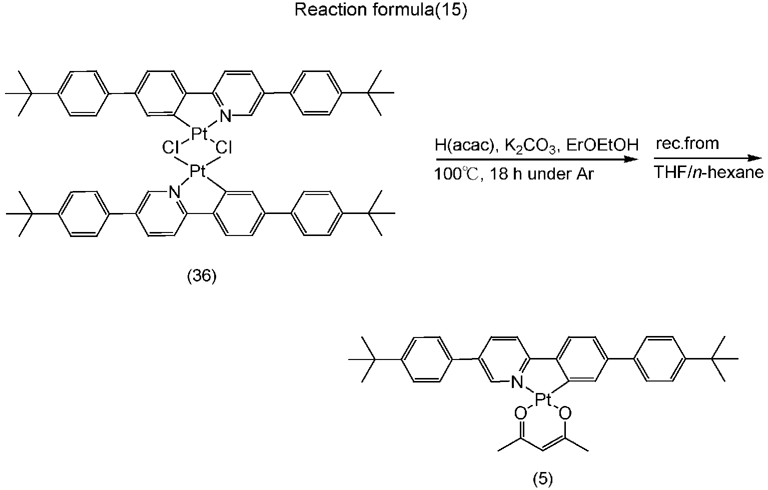

### [Example 5]

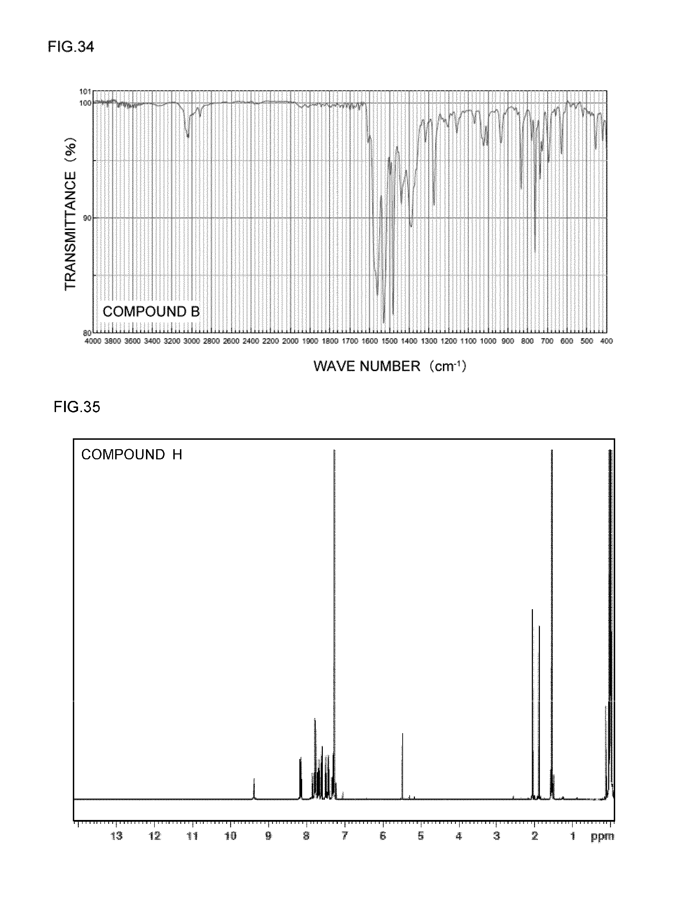

In an example 5,
[acetylacetonate-κO2,κO4] [1-[5-(1,1'-biphenyl)-4-ylpyridyl-κN]-phenyl-κC2] platinum (II) (abbreviated as compound B in some cases) represented by the formula (6) was synthesized as a phosphorescent material, and evaluations were made in the same manner as in the example 1. A NMR chart of the obtained phosphorescent material is shown in FIG. 33, and a FT-IR chart of the obtained phosphorescent material is shown in FIG. 34.

### (1) Providing step 1

That is, as shown in the reaction formula (16), 5-bromo-2-phenylpyridine (1.17 g, 5.0 mmol) represented by the formula (26) was placed in a container with a stirrer, 2-{(1,1'-biphenyl)-4-yl}-4,4,5,5-tetramethyl-1,3,2-dioxabor olane (1.40 g, 5.0 mmol) represented by the formula (37), tetrakis(triphenylphosphine) palladium (0) (0.25 g, 0.22 mmol), potassium carbonate (3.18 g, 23 mmol), 75 ml of THF and 15 ml of water were then added under a nitrogen atmosphere, and the mixture was degassed, and then heated and stirred at 60°C for 10 hours.

Then, the organic solvent was evaporated from the resulting reaction solution, and the resulting precipitate was then filtered. The resulting residue solid was washed with water and methanol, and then recrystallized using dichloromethane/n-hexane to obtain, as a white crystal, 5-((1,1'-biphenyl)-4-yl)-2-phenylpyridine (1.33 g, 4.31 mmol) represented by the formula (38).

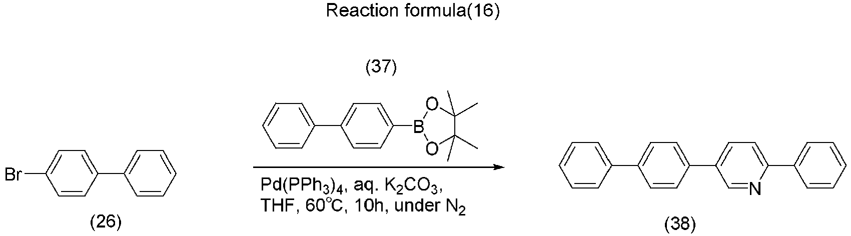

### (2) Acetylacetonating step

Then, as shown in the reaction formula (17), [acetylacetonate-κO2,κO4] [1-[5-(1,1'-biphenyl)-4-ylpyridyl-κN]phenyl-κC2] platinum (II) represented by the formula (6) was synthesized from 5-((1,1'-biphenyl)-4-yl)-2-phenylpyridine represented by the formula (38).

That is, into a container with a stirrer were added 5-((1,1'-biphenyl)-4-yl)-2-phenylpyridine (0.61 g, 2.0 mmol) represented by the formula (38), sodium acetate (0.17 g, 2.0 mmol) and
chloro[acetylacetonate-κO2,κO4][dimethylsulfoxide-κS] platinum (II) (0.81 g, 2.0 mmol), 200 ml of dehydrated methanol and 20 ml of dehydrated THF were further added, and the mixture was heated and stirred at 68°C for 5 days under a nitrogen stream.

Then, the resulting reaction solution was evaporated, and the residue was fractionated by column chromatography (developing solvent: dichloromethane/n-hexane = 1/1 (v/v)), and recrystallized using dichloromethane/n-hexane to obtain, as an orange crystal,
[acetylacetonate-κO2,κO4] [1-[5-(1,1'-biphenyl)-4-ylpyridyl-κN]phenyl-κC2] platinum (II) (0.21 g, 0.35 mmol) represented by the formula (6).

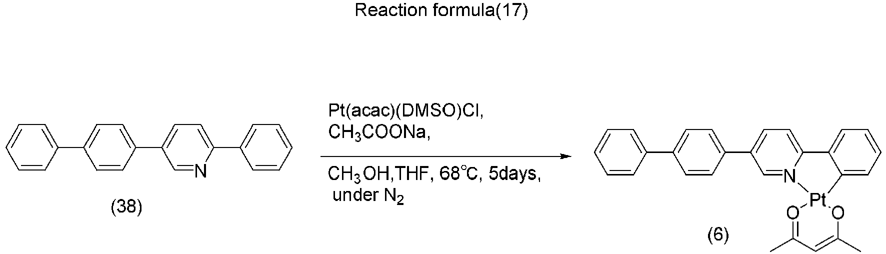

### [Example 6]

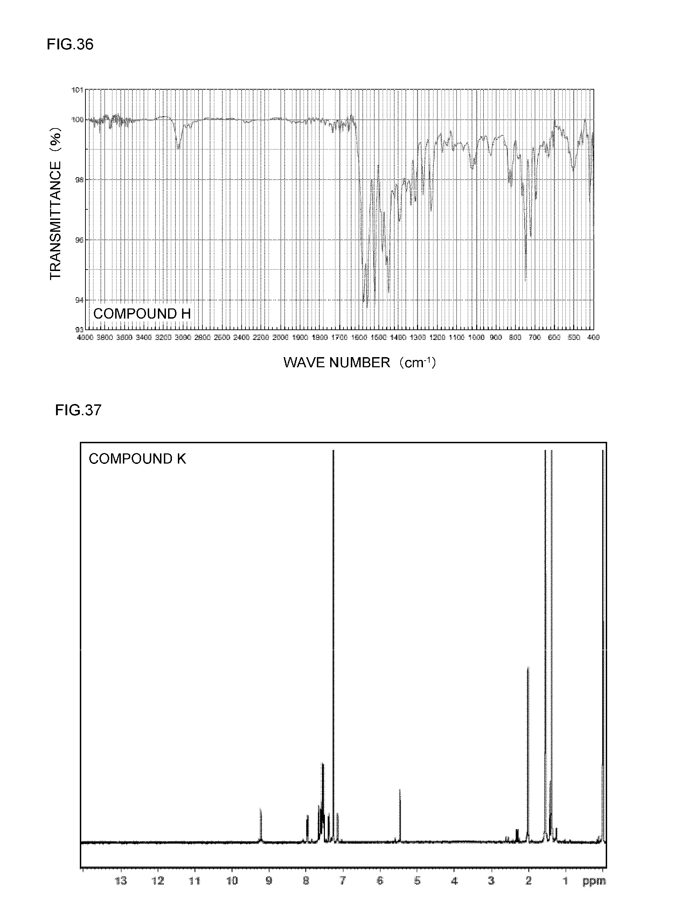

In an example 6,
[acetylacetonate-κO2,κO4] [1-[5-(4-(1,1'-biphenyl))pyridyl-κ -N]-4-(N-carbazolyl)phenyl-κC2] platinum (II) (referred to as compound H in some cases) represented by the formula (7) was synthesized as a phosphorescent material, and evaluations were made in the same manner as in the example 1. A NMR chart of the obtained phosphorescent material is shown in FIG. 35, and a FT-IR chart of the obtained phosphorescent material is shown in FIG. 36.

### (1) Providing step 1

That is, as shown in the reaction formula (18), 2,5-dibromopyridine (2.37 g, 10 mmol) represented by the formula (39) was placed in a container with a stirrer, 2-{4-(N-carbazolylphenyl)}-4,4,5,5-tetramethyl-1,3,2-dioxab -orolane (0.55 g, 2.0 mmol) represented by the formula (40), tetrakis(triphenylphosphine) palladium (0) (0.10 g, 0.04 mmol), potassium carbonate (1.27 g, 9.2 mmol), 30 ml of THF and 6 ml of water were then added under a nitrogen atmosphere, and the mixture was degassed, and then heated and stirred at 60°C for 10 hours.

Then, the solvent was evaporated from the resulting reaction solution, and the resulting precipitate was then filtered, and washed with water and methanol.

Then, the resulting solid was fractionated by column chromatography (developing solvent: dichloromethane/n-hexane = 1/1 (v/v)), and recrystallized using dichloromethane/n-hexane to obtain, as a white crystal, 5-bromo-2-{4-(N-carbazolylphenyl)}pyridine (1.52 g, 3.8 mmol) represented by the formula (41).

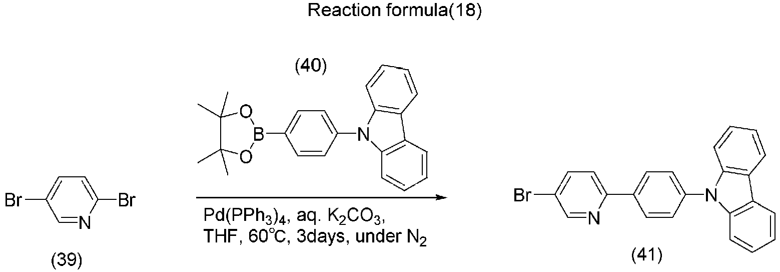

### (2) Providing step 2

Then, as shown in the reaction formula (19), 5-bromo-2-{4-(N-carbazolylphenyl)}pyridine (0.78 g, 2.0 mmol) represented by the formula (41) was placed in a container with a stirrer, 2-{(1,1'-biphenyl)-4-yl}-4,4,5,5-tetramethyl-1,3,2-dioxabor -olane (1.85 g, 5.0 mmol) represented by the formula (37), tetrakis(triphenylphosphine) palladium (0) (0.25 g, 0.22 mmol), potassium carbonate (3.18 g, 23 mmol), 75 ml of THF and 15 ml of water were then added under a nitrogen atmosphere, and the mixture was degassed, and then heated and stirred at 60°C for 3 days.

Then, the solvent was evaporated from the resulting reaction solution, and the resulting precipitate was then filtered.

Then, the resulting residue solid was washed with water and methanol, and then recrystallized using dichloromethane/methanol to obtain, as a white crystal, 5-{4-(1,1'-biphenyl)}-2-{4-(N-carbazolylphenyl)}pyridine (0.88 g, 1.9 mmol) represented by the formula (42).

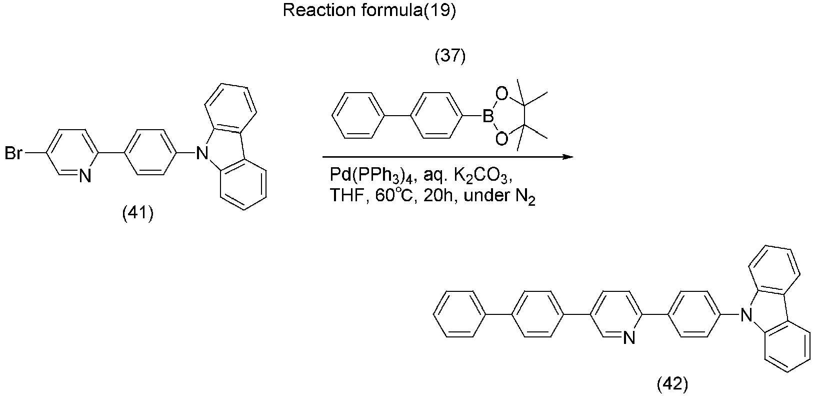

### (3) Providing step 3

Then, as shown in the reaction formula (20), 5-{4-(1,1'-biphenyl)}-2-{4-(N-carbazolylphenyl)}pyridine (0.30 g, 0.64 mmol) represented by the formula (42) was placed in a container with a stirrer, 100 ml of acetic acid and an aqueous solution obtained by dissolving potassium tetrachloroplatinate (0.26 g, 0.64 mmol) in 5 ml of water were then further added under an argon atmosphere, and the mixture was heated and stirred at 100°C for 11 hours.

Then, the reaction solution was filtered, and the solid was then washed with water and methanol to obtain, as a binuclear complex in the form of an ochre powder, di-µ-chlorobis[1-[5-(4-(1,1'-biphenyl))pyridyl-κN]-4-(N-car -bazolyl)phenyl-κC2] diplatinum (II) (0.42 g, 0.30 mmol) represented by the formula (43).

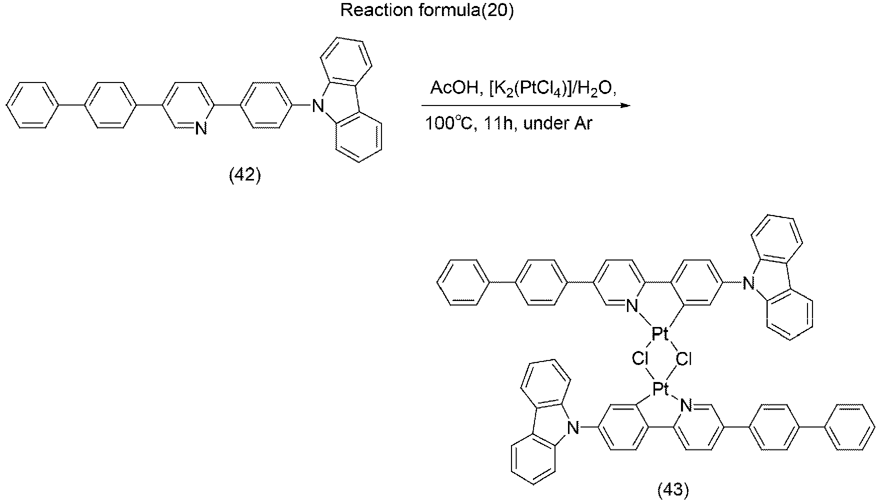

### (4) Providing step 4

Then, as shown in the reaction formula (21), di-µ-chlorobis[1-[5-(4-(1,1'-biphenyl))pyridyl-κN]-4-(N-car -bazolyl)phenyl-κC2] diplatinum (II) (0.33 g, 0.24 mmol) represented by the formula (43) was placed in a container with a stirrer, 13 ml of dimethylsulfoxide was then added under a nitrogen atmosphere, and the mixture was heated and stirred at 190°C for 30 minutes.

Then, the solvent was evaporated from the reaction solution, and the resulting residue was then fractionated by column chromatography (developing solvent: chloroform) to obtain, as an orange solid, chloro[1-[5-(4-(1,1'-biphenyl)pyridyl-κN]-4-(N-carbazolyl)p -henyl-κC2][dimethylsulfoxide-κS] platinum (II) (0.14 g, 0.18 mmol) represented by the formula (44).

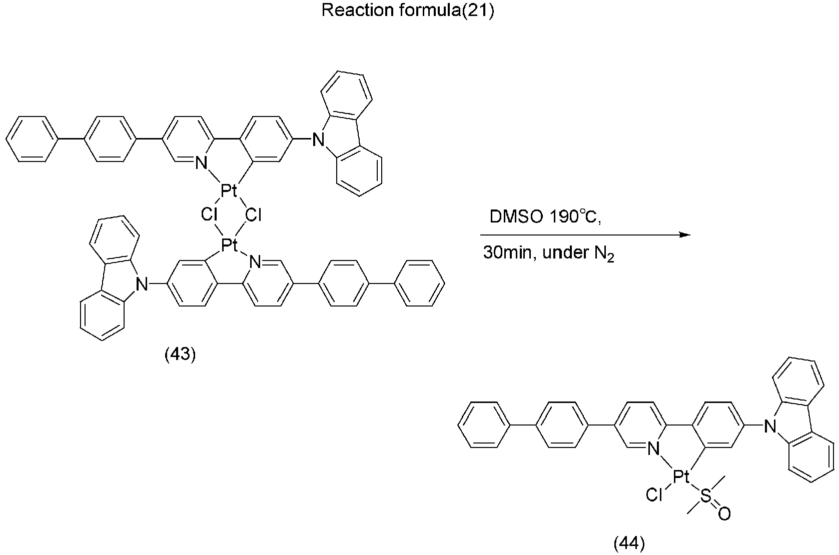

### (5) Acetylacetonating step

Then, as shown in the reaction formula (22), chloro[2-(5-(-4-(N-carbazolylphenyl))pyridyl-κN)phenyl-κC][ dimethylsulfoxide-κS] platinum (II) (0.14 g, 0.18 mmol) represented by the formula (44) was placed in a container with a stirrer, acetylacetonate sodium (0.03 g, 0.03 mmol) and 40 ml of acetone were then further added under a nitrogen stream, and the mixture was stirred at room temperature for 23 hours.

Then, the solvent was evaporated from the resulting reaction solution, and a solid precipitated by adding n-hexane to the concentrated reaction solution was washed with water and methanol.

Then, the resulting solid was fractionated by column chromatography (developing solvent: dichloromethane/n-hexane = 1/1 (v/v)), and recrystallized using dichloromethane/n-hexane to obtain, as a yellow powder, [acetylacetonate-κO2,κO4] [1-[5-(4-(1,1'-biphenyl))pyridyl-κ -N]-4-(N-carbazolyl)phenyl-κC2] platinum (II) (0.060 g, 0.078 mmol) represented by the formula (7).

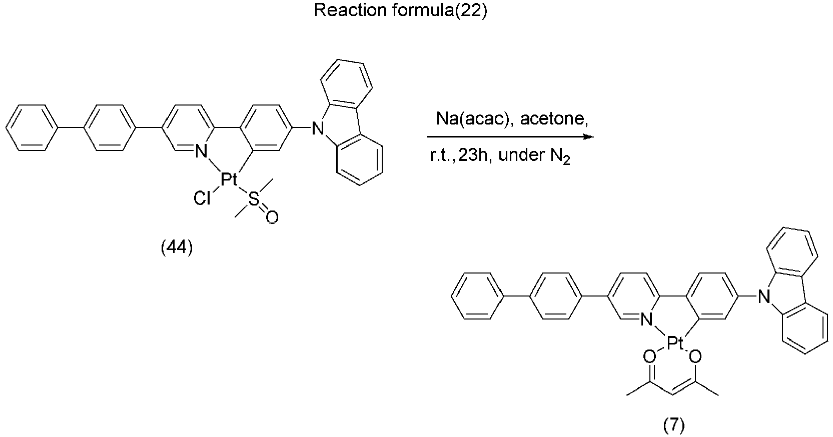

### [Example 7]

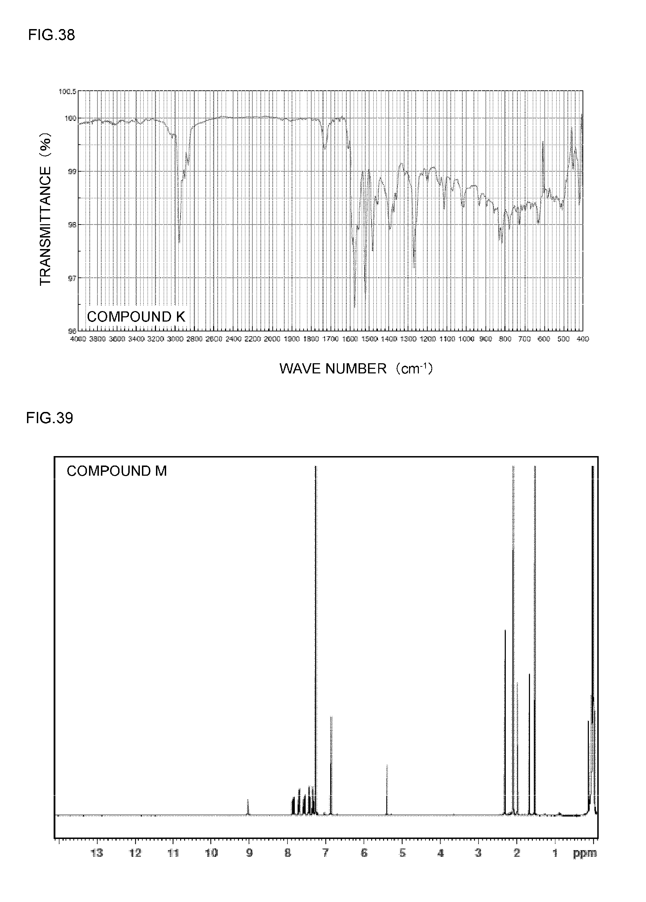

In an example 7,
[acetylacetonate-κO2,κO4][1-[2-(5-(4-tert-butylphenyl))pyri -dyl-κN]-4-tert-butylphenyl)-κC2] platinum (II) (referred to as compound K in some cases) represented by the formula (8) was synthesized as a phosphorescent material, and evaluations were made in the same manner as in the example 1. A NMR chart of the obtained phosphorescent material is shown in FIG. 37, and a FT-IR chart of the obtained phosphorescent material is shown in FIG. 38.

### (1) Providing step 1

That is, as shown in the reaction formula (23), 5-bromo-2-(4-tert-butylphenyl)pyridine (0.82 g, 2.8 mmol) represented by the formula (21) was placed in a container with a stirrer,
2-{1-(4-tert-butylphenyl)}-4,4,5,5-tetramethyl-1,3,2-dioxab -orolane (0.74 g, 2.9 mmol) represented by the formula (31), tetrakis(triphenylphosphine) palladium (0) (0.57 g, 0.49 mmol), 15 ml of a 2 M aqueous potassium carbonate solution and 75 ml of THF were then added under a nitrogen atmosphere, and the mixture was degassed, and then heated and stirred at 60°C for 18 hours.

Then, the solvent was evaporated from the resulting reaction solution, 60 ml of water was then added, and the mixture was extracted with dichloromethane.

Then, the resulting organic layer was washed with a saturated saline solution, and then dried with magnesium sulfate, and the solvent was evaporated.

Further, the resulting residue was fractionated by column chromatography (developing solvent: dichloromethane), and recrystallized using dichloromethane/n-hexane to obtain, as a white powder, 2,5-bis(1-(4-tert-butylphenyl)pyridine (0.42 g, 1.2 mmol) represented by the formula (46).

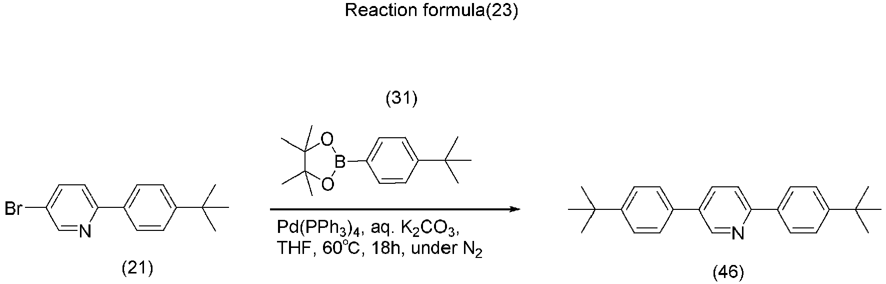

### (2) Providing step 2

Then, as shown in the reaction formula (24), 2,5-bis(1-(4-tert-butylphenyl)pyridine (0.34 g, 1.0 mmol) represented by the formula (46) was placed in a container with a stirrer, 150 ml of acetic acid and an aqueous solution formed by dissolving potassium tetrachloroplatinate (0.42 g, 1.0 mmol) in 10 ml of water were then added under an argon atmosphere, and the mixture was heated and stirred at 110°C for 17 hours.

Then, the solvent was evaporated from the resulting reaction solution, and the resulting residue was then recrystallized using dichloromethane/n-hexane to obtain, as a binuclear complex in the form of an ochre powder, di-µ-chlorobis[1-[2-(5-(4-tert-butylphenyl))pyridyl-κN]-4-t -ert-butylphenyl-κC2] diplatinum (II) (0.80 g, 0.70 mmol) represented by the formula (47).

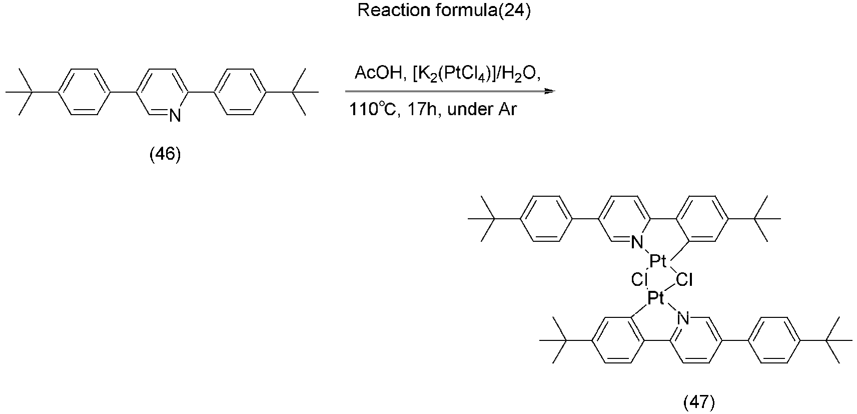

### (3) Acetylacetonating step

Then, as shown in the reaction formula (25), di-µ-chlorobis[1-[2-(5-(4-tert-butylphenyl))pyridyl-κN]-4-t -ert-butylphenyl-κC2] diplatinum (II) (0.19 g, 0.18 mmol) represented by the formula (47) was placed in a container with a stirrer, potassium carbonate (0.26 g, 1.9 mmol) was then added under an argon atmosphere.

Then, a solution obtained by dissolving acetylacetone (0.15 g, 1.5 mmol) in 6 ml of ethylcellosolve was added, and the mixture was then heated and stirred at 100°C for 13 hours.

Then, the solvent was evaporated from the resulting reaction solution, and the resulting solid was then washed with methanol to obtain, an ochre powder, [acetylacetonate-κO2,κO4] [1-[2-(5-(4-tert-butylphenyl))pyri -dyl-κN]-4-tert-butylphenyl-κC2] platinum (II) (0.13 g, 0.20 mmol) represented by the formula (8).

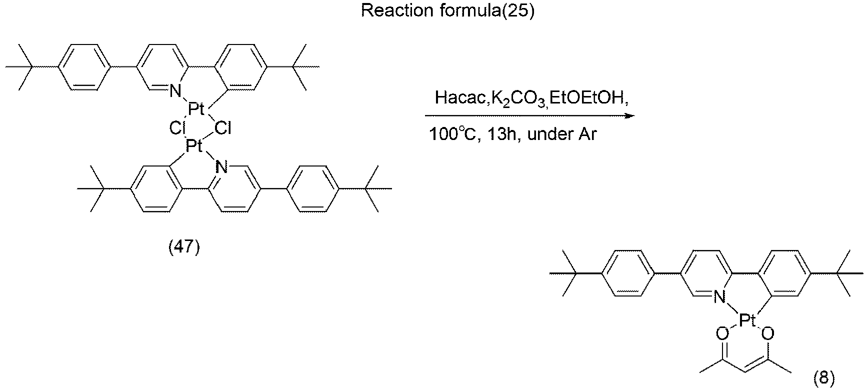

### [Example 8]

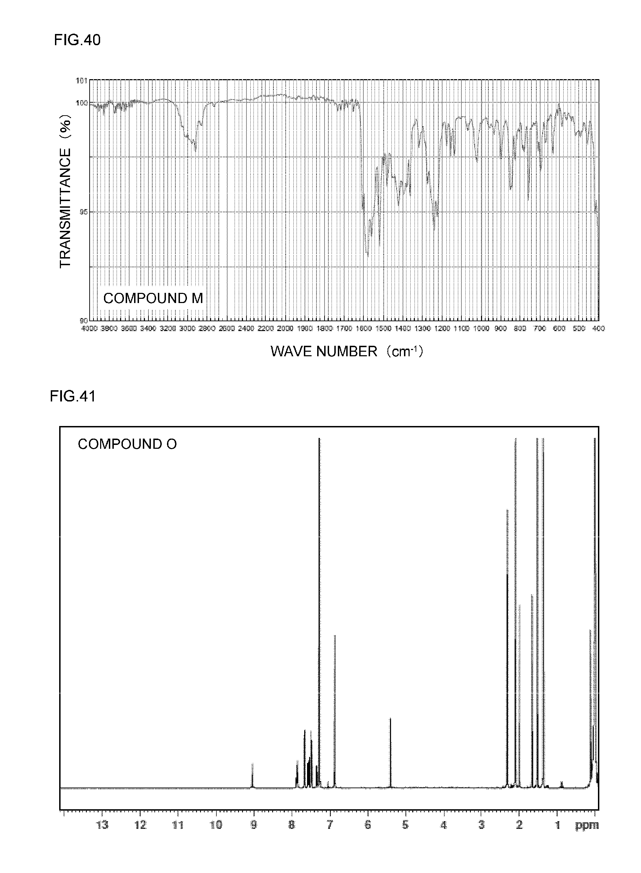

In an example 8,
[acetylacetonate-κO2,κO4][4-[5-(dimethylboryl)pyridyl-κN]-( 1,1'-biphenyl)-4-yl-κC3] platinum (II) (referred to as compound M in some cases) represented by the formula (9) was synthesized as a phosphorescent material, and evaluations were made in the same manner as in the example 1. A NMR chart of the obtained phosphorescent material is shown in FIG. 39, and a FT-IR chart of the obtained phosphorescent material is shown in FIG. 40.

### (1) Providing step 1

That is, as shown in the reaction formula (26), 2-([1,1'-biphenyl]-4-yl)-5-bromopyridine (0.78 g, 2.5 mmol) represented by the formula (48) was placed in a container with a stirrer, 60 ml of dehydrated THF was then added under a nitrogen stream, and the mixture was cooled to -78°C.

Then, 1.7 ml of a n-butyllithium/n-hexane solution at a concentration of 1.6 M was added, the mixture was stirred for 45 minutes, a solution obtained by dissolving dimethylfluoroborane (0.78 g, 2.9 mmol) in 90 ml of dehydrated THF was added, and the mixture was stirred for 1 hour.

Then, the resulting reaction solution was returned to room temperature, and stirred overnight, the organic solvent was then evaporated, dichloromethane and water were further added to the residue, and the mixture was extracted.

Then, the resulting organic layer was dried with anhydrous magnesium sulfate, the organic solvent was then evaporated, and the residue thus obtained was fractionated by column chromatography (developing solvent:
dichloromethane/n-hexane = 1/1 (v/v)) to obtain, as a yellowish white solid,
2-([1,1'-biphenyl]-4-yl)-5-(dimethylboryl)pyridine (0.78 g, 1.6 mmol) represented by the formula (49).

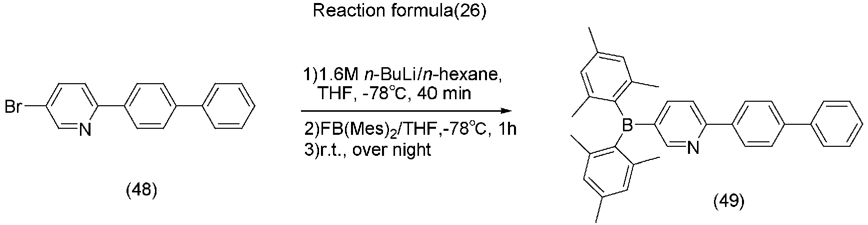

### (2) Acetylacetonating step

Then, as shown in the reaction formula (27), 2-([1,1'-biphenyl]-4-yl)-5-(dimethylboryl)pyridine (0.48 g, 1.0 mmol) represented by the formula (49) was placed in a container with a stirrer, sodium acetate (0.084 g, 1.0 mmol) and chloro[acetylacetonate-κO2,κO4] [dimethysulfoxide-κS] platinum (II) (0.41 g, 1.0 mmol) were then added, 100 ml of dehydrated methanol was further added, and the mixture was heated and stirred at 60°C for 5 days under a nitrogen stream.

The organic solvent was evaporated from the resulting reaction solution, and the resulting residue was then fractionated by column chromatography (developing solvent: dichloromethane/n-hexane = 1/1 (v/v)), and recrystallized using dichloromethane/n-hexane to obtain, as an orange solid, [acetylacetonate-κO2,κO4] [4-[5-(dimethylboryl)pyridyl-κN]-( 1,1'-biphenyl)-4-yl-κC3] platinum (II) (0.092 g, 0.12 mmol) represented by the formula (9).

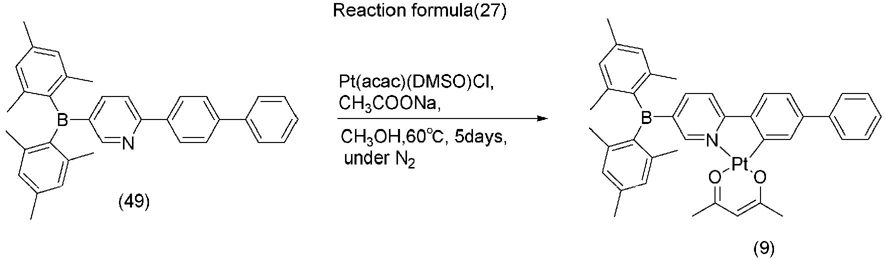

### [Example 9]

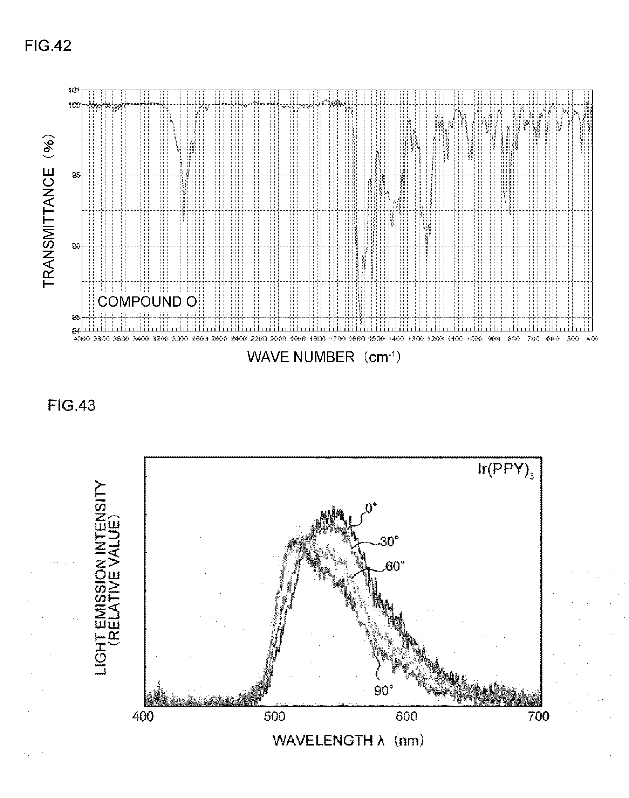

In an example 9,
[acetylacetonate-κO2,κO4] [4-[5-(dimethylboryl)pyridyl-κN]-4 '-(tert-butyl)-(1,1'-biphenyl)-κC3] platinum (II) (referred to as compound O in some cases) represented by the formula (10) was synthesized as a phosphorescent material, and evaluations were made in the same manner as in the example 1. A NMR chart of the obtained phosphorescent material is shown in FIG. 41, and a FT-IR chart of the obtained phosphorescent material is shown in FIG. 42.

### (1) Providing step 1

That is, as shown in the reaction formula (28), 2-(4'-(tert-butyl)-[1,1'-biphenyl]-4-yl)-5-bromopyridine (0.91 g, 2.7 mmol) represented by the formula (34) was placed in a container with a stirrer, 57 ml of dehydrated THF was then added under a nitrogen stream, and the mixture was cooled to -78°C.

Then, 1.7 ml of a n-butyllithium/n-hexane solution at a concentration of 1.6 M was added, the mixture was stirred for 45 minutes, a solution obtained by dissolving dimethylfluoroborane (0.77 g, 2.9 mmol) in 200 ml of dehydrated THF was added, and the mixture was stirred for 1 hour.

Then, the reaction solution was returned to room temperature, and stirred overnight, the organic solvent was then evaporated, dichloromethane and water were added to the residue, and the mixture was extracted.

Then, the resulting organic layer was dried with anhydrous magnesium sulfate, the organic solvent was evaporated, and the residue thus obtained was then fractionated with column chromatography (developing solvent: dichloromethane/n-hexane = 1/1 (v/v)) to obtain, as a yellowish white solid, 2-(4'-(tert-butyl)-[1,1'-biphenyl]-4-yl)-5-(dimethylboryl)p -yridine (0.66 g, 1.2 mmol) represented by the formula (50).

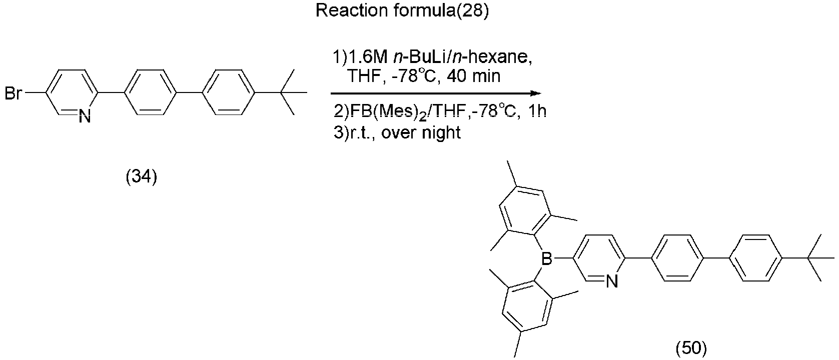

### (2) Acetylacetonating step

Then, as shown in the reaction formula (29), 2-(4'-(tert-butyl)-[1,1'-biphenyl]-4-yl)-5-(dimethylboryl)p -yridine (0.43 g, 0.80 mmol) represented by the formula (50) was placed in a container with a stirrer, potassium acetate (0.078 g, 0.80 mmol) and chloro[acetylacetonate-κO2,κO4] [dimethysulfoxide-κS] platinum (II) (0.33 g, 0.8 mmol) were then added, 100 ml of dehydrated methanol was further added, and the mixture was heated and stirred at 68°C for 5 days under a nitrogen stream.

Then, the organic solvent was evaporated from the resulting reaction solution, and the residue was fractionated by column chromatography (developing solvent: dichloromethane/n-hexane = 1/2 (v/v)), and recrystallized using dichloromethane/n-hexane to obtain, as an orange solid, [acetylacetonate-κO2,κO4] [4-[5-(dimethylboryl)pyridyl-κN]-4 '-(tert-butyl)-(1,1'-biphenyl)-κC3] platinum (II) (0.30 g, 0.36 mmol) represented by the formula (10).

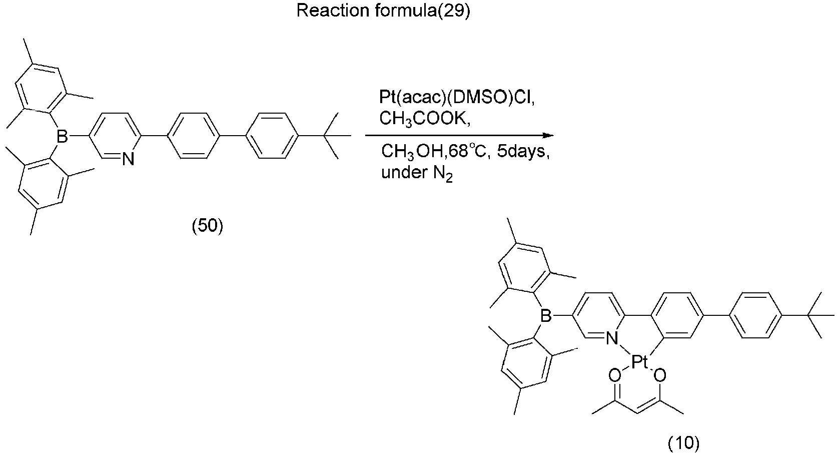

### [Comparative Example 1]

In a comparative example 1, polarizability and the like were evaluated in the same manner as in the example 1 except that the phosphorescent material was changed to tris(2-phenylpyridine) iridium (Ir(PPY)₃) represented by the formula (A), a commercially available phosphorescent compound.

An organic EL element was prepared in the same manner as in the example 2, and external quantum efficiency was measured and found to be about 12%.

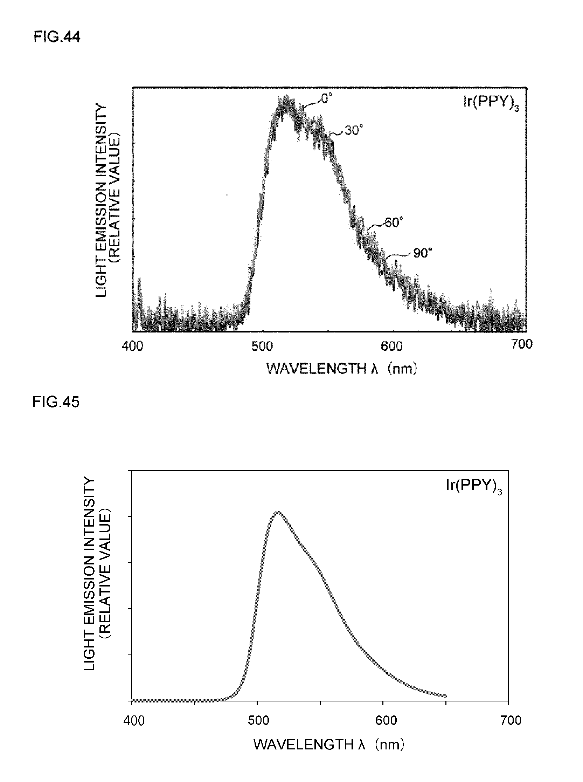

For Ir(PPY)₃, a phosphorescent emission spectrum obtained by the measuring device shown in FIG. 3 is shown in FIG. 43, a phosphorescent emission spectrum obtained by the measuring device shown in FIG. 4 is shown in FIG. 44, and a phosphorescent emission spectrum obtained with the wavelength of excitation light set at 337 nm is shown in FIG. 45.

**Table1**

| | Phosphorescent material | Polarizability | Non-polarizability | Internal quantum efficiency |
|---|---|---|---|---|
| Example1 | Formula(2) b2Pt | Very good | Very good | 51 |
| Example2 | Formula(3) e1Pt | Very good | Very good | 50 |
| Example3 | Formula(4) f1Pt | Fair | Very good | 40 |
| Example4 | Formula(5) c2Pt | Fair | Very good | 34 |
| Example5 | Formula(6) Compound B | Very good | Very good | 50 |
| Example6 | Formula(7) Compound H | Good | Very good | 48 |
| Example7 | Formula(8) Compound K | Good | Very good | 67 |
| Example8 | Formula(9) Compound M | Fair | Very good | 69 |
| Example9 | Formula(10) Compound O | Fair | Very good | 70 |
| Comparative Example1 | Formula(A) Ir(PPY)₃ | Bad | Very good | 97 |

| | | | | |
|---|---|---|---|---|
| * The formula numbers/formula symbols and abbreviations in the column of the phosphorescent material correspond to the formula numbers and the like of the compounds described in the detailed description. | | | | |

As described in detail above, according to the present invention, there can be obtained a phosphorescent material which is excellent in horizontal orientation and the like when formed into a film, a process for efficiently producing the phosphorescent material, and a light emitting element (organic electroluminescence element) using the phosphorescent material.

According to the present invention, the polarizability, stability, light emission quantum efficiency and the like of the phosphorescent material can be appropriately adjusted by introducing various kinds of substituents at the ends of the phosphorescent material.

Accordingly, it is expected that when the phosphorescent material or the like of the present invention is used in the light emitting layer of the organic EL element or the like, light emission of a high luminance and long life with extremely high external quantum efficiency can be achieved, as compared to heretofore, as shown, for example, in the organic EL element of the example 2.

In addition, the phosphorescent material of the present invention is excellent in dispersibility in the host material, and can be mixed in a wide range of amounts, for example 0.1 to 20% by weight based on the total amount of the light emitting layer.

Accordingly, uniform and long-life phosphorescence can be stably obtained in a light emitting element such as an organic EL element not only when the phosphorescent material is mixed in a relatively small amount but also when the phosphorescent material is mixed in a relatively large amount.

### REFERENCE SIGNS LIST

10,10': substrate (glass substrate)
12,12': phosphorescent material (molecule of phosphorescent material)
13,13': light emitting layer
14,14': host material (molecule of host material)
16,16': phosphorescence
18,18': measurement sample
20,20a,20b: laser light (N₂ laser light)
22,22': ND filter
24,24',30,30': lens
26,26': stop
28,28': optical filter
32,32': rotary polarizing filter
34,34': fiber scope
36,36': sample stage
50,50': measuring device
110,111,112,113,114,115: organic EL element
100: transparent substrate
102: anode
103: hole transport layer
103a: hole injection layer
104: light emitting layer
105: hole blocking layer
106: electron transport layer
107: cathode
107a: electron injection layer

## Claims

1. A phosphorescent material represented by the following general formula (1), wherein the phosphorescent material comprises a straight-chain conjugated structure, a 2-phenylpyridine ligand, a central metal and a β-diketone-type ligand, 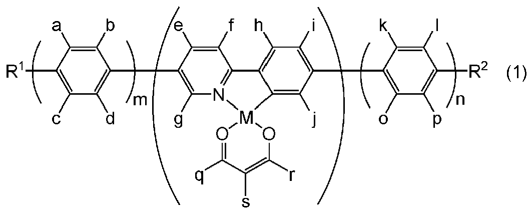 (In the general formula (1), end substituents R¹ and R² are each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group, substituents a to l and o to s are each independently a hydrogen atom, a substituted or unsubstituted alkyl group having 1 to 20 carbon atoms, a substituted or unsubstituted aryl group having 6 to 20 carbon atoms, or a halogen atom, the central metal M is platinum (Pt), iridium (Ir), nickel (Ni), copper (Cu) or gold (Au), repetition numbers m and n are each independently an integer of 0 to 4, and m + n is an integer of 1 or greater).

2. The phosphorescent material according to claim 1, wherein the R¹ and R², or any one thereof, are a tertiary butyl group.

3. The phosphorescent material according to claim 1 or 2, wherein a Stokes shift is set to a value of 130 nm or more.

4. The phosphorescent material according to any one of claims 1 to 3, wherein the phosphorescent material represented by the general formula (1) is at least one of compounds represented by the following structural formulae (2) to (10).

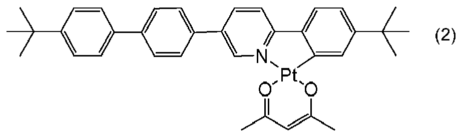

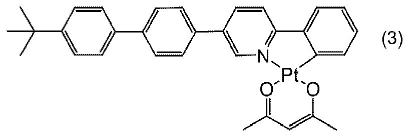

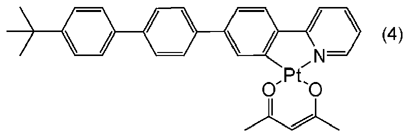

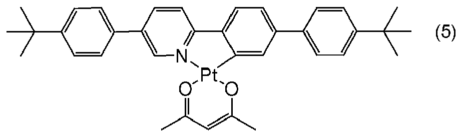

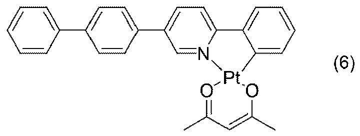

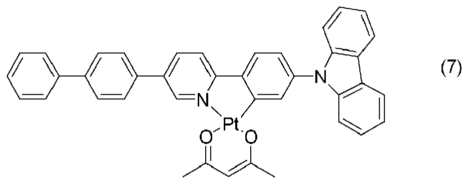

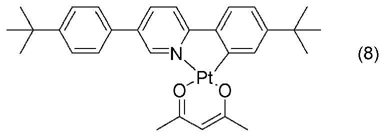

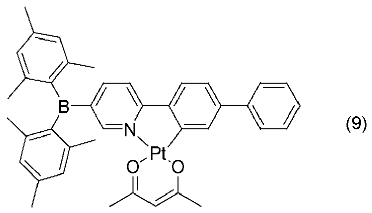

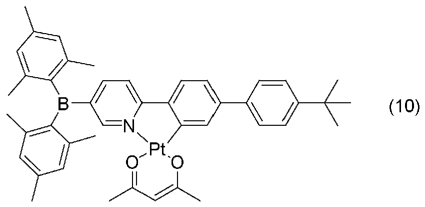

5. A process for producing a phosphorescent material represented by the following general formula (1) and having a straight-chain conjugated structure, a 2-phenylpyridine ligand, a central metal and a β-diketone-type ligand, wherein the process comprises steps of: providing a binuclear complex represented by the following general formula (11); and acetylacetonating the binuclear complex, 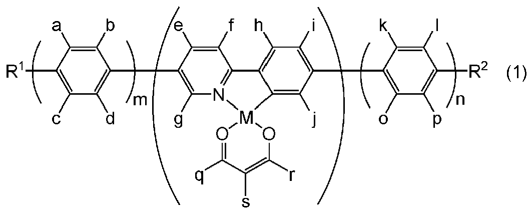 (In the general formula (1), end substituents R¹ and R² are each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group, substituents a to l and o to s are each independently a hydrogen atom, a substituted or unsubstituted alkyl group having 1 to 20 carbon atoms, a substituted or unsubstituted aryl group having 6 to 20 carbon atoms, or a halogen atom, the central metal M is platinum (Pt), iridium (Ir), nickel (Ni), copper (Cu) or gold (Au), repetition numbers m and n are each independently an integer of 0 to 4, and m + n is an integer of 1 or greater), 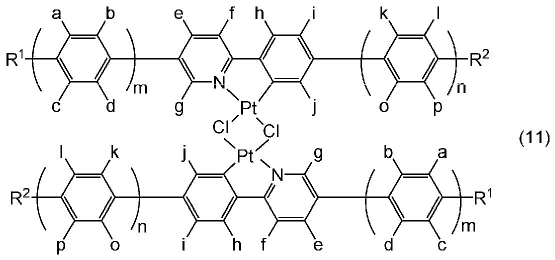 (In the general formula(11), a plurality of end substituents R¹ and R² is each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group, a plurality of substituents a to l and o to s is each independently a hydrogen atom, a substituted or unsubstituted alkyl group having 1 to 20 carbon atoms, a substituted or unsubstituted aryl group having 6 to 20 carbon atoms, or a halogen atom, a plurality of central metals M is each independently platinum (Pt), iridium (Ir), nickel (Ni), copper (Cu) or gold (Au), a plurality of repetition numbers m and n is each independently an integer of 0 to 4, and m + n is an integer of 1 or greater).

6. The process for producing a phosphorescent material according to claim 5, wherein the process comprises a step of obtaining the binuclear complex represented by the general formula (11) by synthesizing a straight-chain aryl compound including a pyridine structure, followed by performing a heat treatment in the presence of potassium tetrachloroplatinate and acetic acid.

7. A light emitting element which comprises a light emitting layer or a plurality of organic thin film layers including the light emitting layer between a pair of electrodes including an anode and a cathode, wherein the light emitting layer comprises a host material as a main component and, as a dopant material, a phosphorescent material represented by the following general formula (1) and having a straight-chain conjugated structure, a 2-phenylpyridine ligand, a central metal and a β-diketone-type ligand, 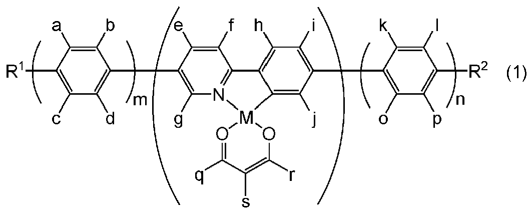 (In the general formula (1), end substituents R¹ and R² are each independently a hydrogen atom, a substituted or unsubstituted alkyl group having 1 to 20 carbon atoms, a substituted or unsubstituted aryl group having 6 to 20 carbon atoms, a halogen atom, a substituted or unsubstituted boryl group, or a substituted or unsubstituted amino group, substituents a to l and o to s are each independently a hydrogen atom, a substituted or unsubstituted alkyl group having 1 to 20 carbon atoms, a substituted or unsubstituted aryl group having 6 to 20 carbon atoms, or a halogen atom, the central metal M is platinum (Pt), iridium (Ir), nickel (Ni), copper (Cu) or gold (Au), repetition numbers m and n are each independently an integer of 0 to 4, and m + n is an integer of 1 or greater).

8. The light emitting element according to claim 7, wherein the mixed quantity of the phosphorescent material is set to a value ranging from 0.1 to 20% by weight based on the total amount of the light emitting layer.
